# EUROPÄISCHE PATENTANMELDUNG

(11) **EP 3 741 316 A1**
(43) Veröffentlichungstag der Anmeldung: **25.11.2020**
(21) Anmeldenummer: 20168244.0
(22) Anmeldetag: 06.04.2020
(51) Int. Cl.: A61B 17/32, A61B 17/14, A61B 17/16, F01B 11/00

(54) **DIFFERENZDRUCKMOTOR UND VERFAHREN ZUM BETREIBEN EINES DIFFERENZDRUCKMOTORS**

(30) Priorität: 22.05.2019 DE 102019113640
(71) Anmelder: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(57) **Zusammenfassung**

Die Erfindung betrifft einen Differenzdruckmotor aufweisend zwei Arbeitskolben (1, 2), eine Stange (5) und einen Hohlraum, der zumindest bereichsweise zylindrisch ist, wobei die Arbeitskolben (1, 2) und die Stange (5) beweglich in dem Hohlraum angeordnet sind, wobei in Wandungen (10) des Hohlraums fünf durchgehende Öffnungen (18, 20, 22, 24, 26) angeordnet sind, einen Ventilkolben (6), wobei der Ventilkolben (6) zwischen den Arbeitskolben (1, 2) gegen die Arbeitskolben (1, 2) beweglich angeordnet ist und der Ventilkolben (6) durch Stöße der Arbeitskolben (1, 2) gegen den Ventilkolben (6) anzutreiben ist und in dem zylindrischen Hohlraum beweglich ist, wobei der Ventilkolben (6) mit den fünf durchgehenden Öffnungen (18, 20, 22, 24, 26) ein Ventil bildet, mit dem eine abwechselnde Beaufschlagung von Arbeitsflächen (3, 4) der Arbeitskolben (1, 2) mit einem ersten Druck und einem zweiten Druck steuerbar ist, wenn der erste Druck und der zweite Druck an drei der fünf durchgehenden Öffnungen (18, 20, 22, 24, 26) angelegt sind, so dass eine periodische Bewegung der Arbeitskolben (1, 2) im Hohlzylinder entsteht, die eine periodische Bewegung des Ventilkolbens (6) antreibt.

Die Erfindung betrifft auch ein chirurgisches Antriebssystem mit einem solchen Differenzdruckmotor, ein medizinisches Lavage-System zum Debridement von Weichgewebe und/oder Knochengewebe aufweisend einen solchen Differenzdruckmotor und eine medizinische Vorrichtung zum Bürsten, Raspeln oder Sägen von Weichgewebe und/oder Knochengewebe mit einem solchen Differenzdruckmotor sowie ein Verfahren zum Betreiben eines Differenzdruckmotors.

## Beschreibung

Die Erfindung betrifft einen Differenzdruckmotor, ein chirurgisches Antriebssystem mit einem solchen Differenzdruckmotor, ein medizinisches Lavage-System zum Debridement von Weichgewebe und/oder Knochengewebe aufweisend einen solchen Differenzdruckmotor und eine medizinische Vorrichtung zum Bürsten, Raspeln oder Sägen von Weichgewebe und/oder Knochengewebe mit einem solchen Differenzdruckmotor sowie ein Verfahren zum Betreiben eines Differenzdruckmotors. Der Differenzdruckmotor ist zum Antrieb von medizinischen Vorrichtungen für das Lavagieren und das Debridement von Weich- und Knochengewebe geeignet. Die mit dem Differenzdruckmotor angetriebenen medizinischen Vorrichtungen sind vorzugsweise aus hygienischen Gründen zum einmaligen Gebrauch bestimmt.

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Das Debridieren kann durch Wundspülung mit sogenannten Lavage-Systemen sowie durch Ausschneiden, Raspeln, Sägen und auch Bürsten erfolgen. Die zum Debridieren verwendeten Vorrichtungen sind nach dem Debridement mit Geweberesten und mit mikrobiellen Keimen kontaminiert. Für eine neue Verwendung müssen diese Instrumente sorgfältig gereinigt und anschließend sterilisiert werden. Dabei muss sich das medizinische Personal vor einer Kontamination beziehungsweise einer Infektion durch Übertragung von mikrobiellen Keimen während der Reinigungsarbeiten schützen. Daher ist es wünschenswert, wenn eine kostengünstige Vorrichtung mit Motorantrieb zum Raspeln, Sägen und Bürsten für septische Revisionen bereitgestellt werden könnte, die nach einmaliger Verwendung ohne aufwendige und nicht gefahrlose Reinigungsschritte mit dem üblichen OP-Abfall zusammen entsorgt werden könnte. Es wäre dann besonders aus Gründen des Ressourcen- und Umweltschutzes aber auch aus Kostengründen sinnvoll, wenn für den Antrieb keine Batterien, Akkumulatoren und Elektromotoren notwendig wären.

In der Medizin sind gegenwärtig mit Druckluft und mit elektrischer Energie betriebene Antriebsvorrichtungen bekannt. Die auf Druckluft basierenden Antriebssysteme enthalten meisten Lamellenmotoren. Problematisch ist, dass unsterile Druckluft mit einem Schlauch zugeführt und nach dem Antrieb des Motors die entspannte, unsterile Luft in einem separaten Schlauch wieder aus dem Operationssaal abgeführt werden muss. Vielfach werden dazu koaxiale Schlauchsystem eingesetzt. Die mit Druckluft betriebenen Antriebe wurden weitgehend durch elektrisch betriebene Antriebssysteme ersetzt. Diese Antriebssysteme enthalten Elektromotoren mit Getrieben und nutzen als Energiequelle üblicherweise Akkumulatoren. Die elektrisch betriebenen Antriebssysteme enthalten Kupfer und weitere Schwermetalle.

Bei Lavage-Systemen werden Sprühstrahlen aus Spülflüssigkeiten erzeugt, die auf die zu reinigenden Gewebeareale auftreffen und eine mechanische Reinigungswirkung auf diese Gewebeareale ausüben. Gepulste Lavage-Systeme sind seit langem bekannt, beispielsweise aus der US 4 583 531 A, der US 4 278 078 A und der US 5 542 918 A.

Bei der Verwendung von Druckluft-getriebenen Lavage-Systemen muss jedoch ein Doppelschlauchsystem verwendet werden, bei dem in einem Schlauch die unsterile Druckluft zugeführt wird und einen zweiten Schlauch mit dem die nach dem Antrieb des Druckluftmotors zumindest teilentspannte unsterile Luft abgeführt wird. Bei den mit Druckluft oder einem anderen komprimierten Gas getriebenen Systemen wird üblicherweise ein Druckgasmotor zum Antrieb eingesetzt. Ein solcher Druckgasmotor ist aus der WO 2012/038003 A1 bekannt. Der dort beschriebene Druckgasmotor hat einen zweiteiligen Kolben mit einem Zwischenraum und einen Durchgang durch einen der Kolbenteile. Dadurch ist der Motor besonders einfach und kostengünstig im Aufbau. Ein Druckgasmotor für eine Sprühdose ist aus der DE 37 24 110 A1 bekannt. Eine mit Druckgas angetriebene Hubkolbenpumpe ist aus der US 4 993 924 A bekannt. Ein Druckgasmotor für ein Lavage-System ist aus der EP 2 873 856 A1 bekannt. Bei diesem Druckgasmotor wird ein Steuerkolben von einem Arbeitskolben über ein Mitnehmerelement bewegt und dadurch werden im Betrieb des Druckgasmotors eine Gaseinlassöffnung und eine Gasauslassöffnung periodisch geöffnet und geschlossen.

Bei einer Vielzahl von Operationen ist ein Absaugen von Wundsekret und Blut notwendig. Für das Absaugen dieser Flüssigkeiten werden Absaugvorrichtungen eingesetzt, die mit Unterdruck betrieben werden. Für den Betrieb dieser Vorrichtungen gibt es dazu in den meisten Operationssälen (OPs) stationäre Vakuumanlagen, die üblicherweise einen Unterdruck von 0,8 bar bis 0,9 bar bereitstellen. Daneben werden auch mobile Vakuumbeziehungsweise Unterdruck-erzeugende Absauganalgen in breitem Umfang eingesetzt.

Es besteht immer der Wunsch nach einem kostengünstiger aufzubauenden Motor. Zudem besteht auch ein Bedürfnis danach, einen Motor bereitzustellen, der mit einer höheren Frequenz und/oder einer größeren Kraft betrieben werden kann.

Die US 5 554 011 A offenbart eine Pumpe mit einem Differenzdruckmotor, bei dem ein Ventilelement durch die Bewegung einer Membran gesteuert wird. An der Membran wird die Arbeit durch den wechselnden Gasdruck geleistet und dadurch die Pumpe angetrieben. Der Aufbau dieses Differenzdruckmotors ist relativ aufwendig und es kann aufgrund der Membran und der notwendigen Verspannung der Membran bei gleichartig aufgebauten Differenzdruckmotoren zu unterschiedlichem Schwingungsverhalten kommen. Zudem besteht bei dem Ventilelement die Gefahr eines Totpunkts, aus dem Heraus der Differenzdruckmotor nicht mehr selber weiterlaufen kann.

Mit dem Patent US 9 861 770 B1 wird ein Vakuummotor vorgeschlagen, bei dem durch Einwirken von Vakuum auf einen Kolben eine Spiralfeder gespannt wird. Am Ende des Spannvorgangs wird ein Impuls auf einen Ventilkolben erteilt, der Belüftungsöffnungen freigibt und kurzzeitig die Vakuumöffnungen verschließt. Die gespannte Spiralfeder kann sich entspannen und treibt den Kolben in die Ausgangslage zurück und treibt damit einen verbundenen Pumpkolben an. Es entsteht eine periodische, lineare, oszillierende Pumpbewegung, die von der Spiralfeder angetrieben wird, wobei das Spannen der Spiralfeder durch eine Druckdifferenz erfolgt.

Aus der US 5 542 918 A ist eine Flüssigkeitspumpe für ein Lavage-System bekannt, das von einem Unterdruck angetrieben wird und bei dem eine Membran von einem hohlförmigen, linear beweglichen und federnd gelagerten Kolben angetrieben wird, in dem ein ebenfalls federnd gelagerter Ventilkolben zum Öffnen und Schließen eines Gaseinlasses zum Zuführen von Luft angeordnet ist. Das System hat den Nachteil, dass das Ventilelement im geöffneten Zustand nur einen geringen freien Strömungsquerschnitt bereitstellen kann. Dadurch ist die mit der Pumpe erzeugbare Kraft begrenzt und es besteht die Gefahr eines Totpunkts, aus dem heraus die Pumpe nicht von alleine wieder startet. Zudem kann es durch die fehlende Führung des Ventilkolbens zu seitlichen Bewegungen des Ventilkolbens und dadurch zu unregelmäßigen Schwingungen des Kolbens kommen.

Die US 2005/0084395 A1 offenbart ein vakuumgetriebenes Lavage-System, das über zwei Zylinder arbeitet. In den Zylindern werden zwei miteinander gekoppelte Kolben von dem Vakuum angetrieben.

In den Patenten EP 2 910 270 B1 und US 9 861 770 B2 wird ein Differenzdruckmotor zum Antrieb einer Pumpe für ein Lavage-System beschrieben. Der Differenzdruckmotor besteht aus einem axial in einem Hohlraum beweglichen Arbeitskolben und einem dahinter angeordneten Rückstellelement. Der Arbeitskolben hat ein Mitnehmerelement, das im Betrieb einen Steuerkolben bewegt. Der Steuerkolben ist ein Hohlzylinder und kann über seine geschlossene Mantelfläche eine Gasauslassöffnung und eine Gaseinlassöffnung verdecken. An die Gasauslassöffnung ist eine Unterdruckquelle wie beispielsweise eine Vakuumpumpe angeschlossen. Das bedeutet, dass beim Betrieb des Motors periodisch die Gasauslassöffnung und die Gaseinlassöffnung verdeckt werden. Bei geöffneter Gasauslassöffnung wird die Luft aus dem Differenzdruckmotor gesaugt, wobei der Arbeitskolben gegen das Rückstellelement bewegt wird. Die Zuluftöffnung ist gleichzeitig durch den Steuerkolben verschlossen. Das Rückstellelement wird gespannt. Dann erteilt das Mitnehmerelement dem Steuerkolben einen Impuls. Der Steuerkolben bewegt sich vom Arbeitskolben weg und öffnet die Gaseinlassöffnung und verschließt gleichzeitig die Gasauslassöffnung. Das Vakuum bricht zusammen und das Rückstellelement bewegt den Arbeitskolben in seine Ausgangslage. Dabei erteilt der Mitnehmer dem Steuerkolben einen Impuls. Der Steuerkolben verschiebt sich in Richtung Arbeitskolben. Die Gaseinlassöffnung wird durch die Mantelfläche des Steuerkolbens verschlossen und die Gasauslassöffnung wird geöffnet. Dann beginnt der Zyklus erneut. Dieser Differenzdruckmotor ist für hohe Pulszahlen von ca. 2000 Pulsen pro Minute zum Betrieb einer Pumpe für ein Lavage-System ausgelegt. Die Hublänge ist relativ kurz und beträgt zwischen 2 und 5 mm. Für das Bürsten, Raspeln oder Sägen werden größere Hublängen und geringe Pulszahlen benötigt. Für die Fertigung des in den Patenten EP 2 910 270 B1 und US 9 861 770 B2 beschriebenen Differenzdruckmotors sind durch Kunststoffpräzisionsspritzgießen gefertigte Bauteile notwendig. Die Montage dieser Teile bedarf einer hohen Sorgfalt.

Für das Sprühen von Flüssigkeiten reichen die mit den Vakuummotoren erreichbaren Kräfte völlig aus. Für den Antrieb von Sägen, Raspeln und Bürsten sind jedoch größere Antriebskräfte notwendig.

In dem Patent US 8 292 909 B1 wird ein doppeltwirkender Vakuummotor beschrieben. Bei diesem wird am Ende der Vorwärts- und der Rückwärtsbewegung des Arbeitskolbens eine Kraft auf einen Ventilkolben durch ein bistabiles Kopplungselement übertragen, wodurch dieser von einem Schaltzustand in einem zweiten Schaltzustand übergeht. Der Ventilkolben verharrt dadurch in einem definierten Schaltzustand während der Bewegung des Arbeitskolbens. Es wird somit durch die jeweilige Endposition des Arbeitskolbens der Schaltzustand des Ventilkolbens definiert.

Die Aufgabe der vorliegenden Erfindung besteht also darin, die Nachteile des Stands der Technik zu überwinden. Insbesondere besteht die Aufgabe der Erfindung in der Entwicklung eines Differenzdruckmotors, der kostengünstig und einfach zu fertigen ist, der zuverlässig arbeitet, vielseitig einsetzbar ist und mit Hilfe einer Druckdifferenz, insbesondere einer Unterdruckquelle oder einer Druckgasquelle, wie sie in Krankenhäusern zur Verfügung stehen, eine ausreichende Leistung erzielt, um Gewebe zu debridieren und/oder Werkzeuge für die Chirurgie anzutreiben. Daher ist es auch Aufgabe der vorliegenden Erfindung, derartige chirurgische Werkzeuge und Lavage-Systeme mit einem solchen Differenzdruckmotor bereitzustellen. Ebenso ist es Aufgabe der vorliegenden Erfindung ein Verfahren zum Betreiben eines Differenzdruckmotors bereitzustellen, wobei das Verfahren die oben zum Differenzdruckmotor genannten Vorteile bietet.

Aufgabe der Erfindung ist es auch, einen extrem vereinfachten, kostengünstigen Differenzdruckmotor zu entwickeln, der mit Vakuum beziehungsweise Unterdruck oder mit Druckgas oder auch mit einer unter Druck stehenden Flüssigkeit angetrieben werden kann und der eine oszillierende, lineare Bewegung erzeugt. Der Differenzdruckmotor soll einfacher aufzubauen und kostengünstiger zu fertigen sein als bekannte Differenzdruckmotoren für den Antrieb eines Lavage-Systems. Der Differenzdruckmotor soll möglichst so beschaffen sein, dass das in Operationssälen über zentrale Vakuumanlagen bereit gestellte Vakuum ausreichend für einen Betrieb ist. Der Hub des Differenzdruckmotors sollte bevorzugt größer als 5 mm sein, um Werkzeuge wie Bürsten oder Sägen damit im medizinischen Bereich wirkungsvoll und ohne weiteres Getriebe betreiben zu können. Der Differenzdruckmotor soll zum Antreiben von Lavage-Systemen und von Vorrichtungen zum Debridement von infiziertem Weich- und Knochengewebe geeignet sein, die aus hygienischen Gründen nur zum einmaligen Gebrauch als sogenannte "disposable devices" (Wegwerfprodukte) bestimmt sind. Der Differenzdruckmotor soll mit Ethylenoxid sterilisierbar sein.

Die Aufgabe der Erfindung besteht auch darin, einen Differenzdruckmotor zu entwickeln, der als einmalig zu verwendender Antrieb für medizinische Sägen, Raspeln, Bürsten und Spülvorrichtungen geeignet ist. Für den Antrieb sollen möglichst keine mechanischen Federn zur Anwendung kommen. Der Differenzdruckmotor soll im Wesentlichen aus Kunststoffen gebildet werden können. Dadurch soll es möglich sein, den Differenzdruckmotor nach seiner Verwendung durch Verbrennung zu entsorgen. Weiterhin soll eine medizinische Antriebseinheit mit dem Vakuummotor entwickelt werden. Diese Antriebseinheit soll zum Betreiben von medizinischen Sägen, Raspeln, Bürsten und Spülvorrichtungen geeignet sein. Diese Antriebseinheit ist insbesondere für septische Revisionen in der Orthopädie bestimmt, bei der Infektionen mit problematischen mikrobiellen Keimen, wie beispielweise MRSA (Methicillin resistenter Staphylococcus aureus), VRSA (Vancomycin resistenter Staphylococcus aureus) ursächlich sind. Konventionelle elektrisch betriebene Antriebseinheiten müssen nach der OP gründlich gereinigt, desinfiziert und sterilisiert werden. Diese Arbeiten sind kostenintensiv und mit einer nicht unerheblichen Infektionsgefahr für das medizinische Personal verbunden. Der zu entwickelnde Differenzdruckmotor und die mit dem Differenzdruckmotor aufgebauten Antriebseinheiten sind daher zum einmaligen Gebrauch bestimmt und sollen durch Verbrennung zusammen mit anderen Krankenhausabfall hygienisch entsorgt werden können. Daher sollen der Differenzdruckmotor und die gesamte medizinische Antriebseinheit im Wesentlichen aus kostengünstigen Kunststoffteilen bestehen, die durch Kunststoffspritzgießen hergestellt werden können und die durch Verbrennung kostengünstig und hygienisch entsorgt werden können. Die Antriebseinheit und der Differenzdruckmotor sollen weiterhin so beschaffen sein, dass eine mikrobielle Kontamination der stationären Vakuumanlagen ausgeschlossen ist.

Die der vorliegenden Erfindung zugrundeliegenden Aufgaben werden gelöst durch einen Differenzdruckmotor aufweisend zwei Arbeitskolben mit je einer Arbeitsfläche, eine Stange, die die Arbeitskolben miteinander verbindet und voneinander beabstandet, einen Hohlraum, der zumindest bereichsweise zylindrisch ist, wobei die Arbeitskolben und die Stange beweglich in dem Hohlraum angeordnet sind, wobei in Wandungen des Hohlraums fünf durchgehende Öffnungen angeordnet sind, einen Ventilkolben, wobei der Ventilkolben zwischen den Arbeitskolben gegen die Arbeitskolben beweglich angeordnet ist und der Ventilkolben durch Stöße der Arbeitskolben gegen den Ventilkolben anzutreiben ist und in dem zylindrischen Hohlraum beweglich ist, wobei der Ventilkolben mit den fünf durchgehenden Öffnungen ein Ventil bildet, mit dem eine abwechselnde Beaufschlagung der Arbeitsflächen der Arbeitskolben mit einem ersten Druck und einem zweiten Druck steuerbar ist, wenn der erste Druck und der zweite Druck an drei der fünf durchgehenden Öffnungen angelegt sind, so dass eine periodische Bewegung der Arbeitskolben und der Stange im Hohlzylinder entsteht, die eine periodische Bewegung des Ventilkolbens antreibt.

Die zwei Arbeitskolben und die Stange sind beweglich gegen den Hohlraum in dem Hohlraum angeordnet.

Bevorzugt ist der Ventilkolben beidseitig durch Stöße der Arbeitskolben gegen den Ventilkolben anzutreiben.

Unter einer Arbeitsfläche eines Arbeitskolbens ist vorliegend die Seite des Arbeitskolbens zu verstehen, auf der durch wechselnde Druckverhältnisse, das heißt durch abwechselndes Wirken des ersten Drucks und des zweiten Drucks, die Arbeit am Arbeitskolben geleistet wird.

Der erste Druck und der zweite Druck werden vorzugsweise über ein fluides Arbeitsmedium vermittelt. Hierzu kann bevorzugt der den Differenzdruckmotor umgebende Luftdruck und ein Vakuum beziehungsweise ein Unterdruck verwendet werden. Alternativ kann aber auch ein Druckgas und der umgebende Luftdruck, ein Vakuum beziehungsweise ein Unterdruck und ein Druckgas sowie theoretisch auch eine unter Druck stehende Flüssigkeit gegenüber einem freien Ausgang für diese Flüssigkeit als fluides Arbeitsmedium zum Bereitstellen des ersten Drucks und des zweiten Drucks verwendet werden. Ein Druckgas kann beispielsweise mit einem Kompressor erzeugt werden oder auch durch Verdampfen von CO₂ aus einer CO₂-Druckgaspatrone erzeugt werden. Ferner kann auch Wasserdampf durch Verdampfen von Wasser durch Einwirken von Wärme erzeugt werden, um einen unter Druck stehenden Wasserdampf als das fluide Arbeitsmedium zu erzeugen. Der Differenzdruckmotor kann also auch als Dampfmaschine verwendet werden. Die Anwendung des Differenzdruckmotors als Vakuummotor ist jedoch insbesondere bei medizinischen Anwendungen bevorzugt, da Anschlüsse hierfür üblicherweise in Operationssälen vorhanden sind.

Bevorzugt sind die fünf Öffnungen axial bezogen auf eine Zylinderachse des Hohlraums nebeneinander angeordnet, besonders bevorzugt entlang einer Reihe. Dann sind die fünf Öffnungen axial nebeneinander aufgereiht, so dass die fünf Öffnungen durch eine mittlere Öffnung, zwei äußere Öffnungen und zwei zu den äußeren Öffnungen benachbarte Öffnungen gebildet sind, wobei die zwei zu den äußeren Öffnungen benachbarten Öffnungen zwischen jeweils einer der äußeren Öffnungen und der mittleren Öffnung angeordnet sind. Die Zylinderachse des Hohlraums ist besonders bevorzugt die Zylinderachse eines Ventilraums, in dem der Ventilkolben beweglich angeordnet ist.

Die Angaben: mittlere Öffnung, äußere Öffnungen und zu den äußeren Öffnungen benachbarte Öffnungen beziehen sich vorliegend auf den Ort, an dem die Öffnungen in den Hohlraum beziehungsweise den Ventilraum einmünden. Diese Anordnung entspricht aber vorzugsweise auch der aus der Wandung des Hohlraums nach außen mündenden Anordnung der durchgehenden Öffnungen. Die durchgehenden Öffnungen können dann einfach den kürzesten Weg gerade durch die Wandung laufen.

Ein Zylinder beziehungsweise eine zylindrische Geometrie im Sinne der vorliegenden Erfindung ist, wie auch nach der allgemeinen Definition, ein von zwei parallelen, ebenen, kongruenten Flächen (Grund- und Deckfläche) und einer Mantelfläche beziehungsweise Zylinderfläche begrenzter Körper, wobei die Mantelfläche von parallelen Geraden gebildet wird. Das heißt, der Zylinder entsteht durch Verschiebung einer ebenen Fläche entlang einer Geraden, die nicht in dieser Ebene liegt. Die Höhe und die Achse des Zylinders ist gegeben durch den Abstand der beiden Ebenen, in denen Grund- und Deckfläche liegen.

Sind die Geraden senkrecht zu Grund- und Deckfläche, spricht man von einem geraden Zylinder. Die gerade zylindrische Geometrie des Innenraums ist erfindungsgemäß bevorzugt. Ein gerader Kreiszylinder stellt im Sinne der vorliegenden Erfindung also nur einen Spezialfall einer zylindrischen Geometrie dar, ist aufgrund der einfacheren Fertigung aber ebenfalls bevorzugt.

Vorliegend wird unter einer axialen Richtung die Richtung bezogen auf die Zylinderachse des zumindest bereichsweise zylindrischen Hohlraums verstanden.

Bevorzugt ist der Ventilkolben ein rotationssymmetrischer Körper, der entlang seiner Symmetrieachse beweglich in dem Hohlraum angeordnet ist.

Es kann bevorzugt vorgesehen sein, dass der Hohlraum durch zwei geschlossene Stirnflächen begrenzt ist. Dabei kann vorgesehen sein, dass die Stange durch eine oder zwei Aussparungen in der Stirnfläche oder den Stirnflächen aus dem Hohlraum herausragt. Die Stange kann dazu durch einen der Arbeitskolben oder durch beide Arbeitskolben hindurchgeführt sein.

Bevorzugt kann vorgesehen sein, dass der Differenzdruckmotor zur einmaligen Verwendung vorgesehen ist. Hierzu kann der Differenzdruckmotor im Wesentlichen aus verbrennbarem Kunststoff bestehen.

Es kann auch vorgesehen sein, dass der Hohlraum zumindest im Bereich des Hubraums der beiden Arbeitskolben und in einem von Ventilkolben überfahrbaren Ventilraum zylindrisch geformt ist.

Es kann auch vorgesehen sein, dass der Hohlraum zwei zylindrische Arbeitsräume aufweist, in denen die zwei Arbeitskolben beweglich angeordnet sind.

Es kann bevorzugt vorgesehen sein, dass der Differenzdruckmotor durch Druckgas und/oder Vakuum antreibbar ist.

Wenn der Differenzdruckmotor mit einem Vakuum oder einem Unterdruck betrieben werden kann, kann er als Vakuummotor bezeichnet werden. Wenn der Differenzdruckmotor mit einem Druckgas beziehungsweise einem Überdruck betrieben werden kann, kann er als Druckgasmotor bezeichnet werden.

Mit der vorliegenden Erfindung kann auch vorgesehen sein, dass die Arbeitskolben den Hohlraum in zumindest drei voneinander getrennte Bereiche unterteilt.

Hierdurch werden ein effizientes Arbeiten und eine hohe Leistung des Differenzdruckmotors ermöglicht.

Des Weiteren kann vorgesehen sein, dass der Hohlraum einen ersten Arbeitsraum und einen zweiten Arbeitsraum aufweist, in denen die Arbeitskolben beweglich angeordnet sind, und einen zwischen den Arbeitsräumen angeordneten Ventilraum aufweist, wobei die fünf durchgehenden Öffnungen in jeder Stellung der Arbeitskolben im Ventilraum angeordnet sind.

Hiermit wird eine Bewegung des Ventilkolbens in dem Ventilraum ermöglicht, wobei in dem Ventilraum die Bewegung des Ventilkolbens nicht durch wechselnde Druckverhältnis oder durch das Leisten von Arbeit beeinträchtigt wird.

Bevorzugt haben der erste Arbeitsraum und der zweite Arbeitsraum einen größeren Querschnitt als der Ventilraum.

Der Ventilraum ist vorzugsweise von den den Arbeitsflächen gegenüberliegenden Seiten der Arbeitskolben und einer zumindest bereichsweise zylindrischen Innenwand begrenzt, die den Hohlraum bereichsweise begrenzt.

Der Ventilkolben ist dann im Ventilraum angeordnet, vorzugsweise auf der Stange und gegen die Stange beweglich angeordnet.

Jeder der Arbeitsräume kann dann mit einer der fünf Öffnungen gasdurchlässig verbunden sein beziehungsweise werden, so dass mit dem Ventil der Druck in den Arbeitsräumen gesteuert werden kann.

Bevorzugt kann vorgesehen sein, dass alle freien Bereiche des Ventilraums gasdurchlässig miteinander verbunden sind. Hierdurch wird verhindert, dass die Bewegung des Ventilkolbens durch einen Aufbau von Gaspolstern im Ventilraum behindert wird. Hierzu kann insbesondere vorgesehen sein, dass zwei Stirnseiten des Ventilkolbens im Hohlraum gasdurchlässig miteinander verbunden sind.

Gemäß einer bevorzugten Weiterbildung der vorliegenden Erfindung kann vorgesehen sein, dass der axiale Abstand einer ersten Stirnfläche des Ventilkolbens zur Rückseite des benachbarten Arbeitskolbens kleiner oder gleich ist, wie der Abstand der Arbeitsfläche dieses Arbeitskolbens zu einer ersten Stirnfläche des Hohlraums, und der axiale Abstand einer zweiten Stirnfläche des Ventilkolbens zur Rückseite des benachbarten Arbeitskolbens kleiner oder gleich ist, wie der Abstand der Arbeitsfläche dieses Arbeitskolbens zu einer zweiten, der ersten Stirnfläche gegenüberliegenden Stirnfläche des Hohlraums.

Hiermit wird sichergestellt, dass die Hub-Bewegung der Arbeitskolben dazu ausreicht, um den Ventilkolben zu erreichen und dadurch anzustoßen und damit das Ventil anzutreiben.

Es kann auch vorgesehen sein, dass die Arbeitskolben und die Stange linear beweglich, insbesondere axial beweglich bezogen auf eine Symmetrieachse der Arbeitskolben und/oder der Stange, in dem zumindest bereichsweise zylindrischen Hohlraum angeordnet sind.

Hierdurch wird eine periodische Linearbewegung erzeugt, die direkt für medizinische Zwecke wie Sägen, Raspeln, Bürsten oder Pumpen verwendet werden kann.

Bevorzugt kann auch vorgesehen sein, dass der Ventilkolben mit den fünf Öffnungen ein 2/5-Wegeventil bildet, bevorzugt ein 2/5-Wege-lmpuls-Ventil bildet.

Ein 2/5-Wegeventil wird auch als 2-Position-5-Wegeventil bezeichnet. Derartige 2/5-Wegeventile sind für den erfindungsgemäßen Differenzdruckmotor besonders gut geeignet und ermöglichen eine stabile und zuverlässige Steuerung der Druckverhältnisse im Differenzdruckmotor.

Des Weiteren kann vorgesehen sein, dass das Ventil eine alternierende Beaufschlagung der Arbeitskolben mit Vakuum oder einem Druckgas und mit umgebender Atmosphäre steuert.

Hiermit wird mit Hilfe des Ventils eine periodische Bewegung des Differenzdruckmotors erzeugt.

Bevorzugte erfindungsgemäße Differenzdruckmotoren können sich auch dadurch auszeichnen, dass die Arbeitsflächen der Arbeitskolben voneinander abgewandt ausgerichtet sind.

Hierdurch kann der Raum, in dem sich der Ventilkolben bewegt, von den Arbeitsräumen, in denen die Leistung an den Arbeitskolben geleistet wird, räumlich getrennt werden, so dass ein direkter Einfluss der Druckverhältnisse in den Arbeitsräumen auf die Bewegung des Ventilkolbens ausgeschlossen werden kann.

Bevorzugt kann ferner vorgesehen sein, dass der Ventilkolben auf der Stange axial verschiebbar angeordnet ist.

Hierdurch wird die Lagerung des Ventilkolbens und damit die Bewegung des Ventilkolbens stabilisiert.

Gemäß einer bevorzugt Weiterbildung der vorliegenden Erfindung kann vorgesehen sein, dass der Ventilkolben eine Hülse mit drei umlaufenden Nuten ist, die durch umlaufende Stege voneinander getrennt sind, wobei die Nuten zumindest so breit sind, wie der auf die Hülse bezogene axiale Abstand zweier axial benachbarter Öffnungen der fünf Öffnungen, so dass bei geeigneter Stellung der Hülse im Hohlraum zwei axial benachbarte Öffnungen in die gleiche Nut der Hülse im Hohlraum münden, und wobei bevorzugt die Stege zumindest in axialer Richtung so breit sind wie die Mündungen in den Hohlraum der beiden Öffnungen der fünf Öffnungen, die zu den äußersten Öffnungen benachbart sind.

Hiermit wird sichergestellt, dass der Ventilkolben in jeder Lage zur Steuerung des Ventils geeignet ist.

Des Weiteren kann vorgesehen sein, dass eine mittlere Öffnung der fünf Öffnungen mit einem Vakuumanschluss oder einem Druckluftanschluss verbunden ist und zwei äußere Öffnungen nach außen zur Umgebung des Differenzdruckmotors offen sind oder zwei äußere Öffnungen der fünf Öffnungen mit einem Vakuumanschluss oder einem Druckluftanschluss verbunden sind und eine mittele Öffnung nach außen zur Umgebung des Differenzdruckmotors offen sind.

Hierdurch ist der Differenzdruckmotor zur Verwendung mit einem Unterdruck beziehungsweise Vakuum verwendbar oder zur Verwendung mit einem Überdruck beziehungsweise einem Druckgas verwendbar. Als zweiter Druck wird dabei immer der den Differenzdruckmotor umgebende Normaldruck beziehungsweise Umgebungsdruck verwendet.

Es kann zu dem gleichen Zweck vorgesehen sein, dass eine mittlere Öffnung der fünf Öffnungen mit einem Vakuumanschluss oder einem Druckluftanschluss verbunden ist oder zwei äußere Öffnungen der fünf Öffnungen mit einem Vakuumanschluss oder einem Druckluftanschluss verbunden sind.

Des Weiteren kann vorgesehen sein, dass eine zu einer äußeren Öffnung benachbarte Öffnung der fünf Öffnungen mit einem durch einen ersten Arbeitskolben der zwei Arbeitskolben begrenzten ersten Arbeitsraum druckleitend verbunden ist und eine andere zu einer äußeren Öffnung benachbarte Öffnung mit einem durch einen zweiten Arbeitskolben der zwei Arbeitskolben begrenzten zweiten Arbeitsraum druckleitend verbunden ist.

Hiermit können die benachbarten Öffnungen der fünf Öffnungen durch eine einfache breite Nut in der Oberfläche des Ventilkolbens miteinander verbunden werden und das Ventil kann so auf einfache Weise die Druckverhältnisse steuern.

Ferner kann vorgesehen sein, dass bei Anlegen eines Vakuums an einem dafür vorgesehenen Vakuumanschluss des Differenzdruckmotors die beiden Arbeitskolben durch periodische Wechsel der Einwirkung von Vakuum und der umgebenden Atmosphäre eine oszillierende Bewegung der Stange bewirken oder bei Einspeisen von Druckgas an einem dafür vorgesehenen Druckgasanschluss des Differenzdruckmotors die beiden Arbeitskolben durch periodische Wechsel der Einwirkung von Druckluft und der umgebenden Atmosphäre eine oszillierende Bewegung der Stange bewirken, wobei in beiden Fällen die Arbeitskolben periodisch den Ventilkolben beidseitig anstoßen und so eine axiale periodische Bewegung des Ventilkolbens verursachen, wodurch ein Umschalten des Ventils erfolgt.

Hierdurch wird eine einfache und sichere Steuerung des Differenzdruckmotors erreicht.

Gemäß einer Weiterbildung der vorliegenden Erfindung kann vorgesehen sein, dass in einer ersten Stellung des Ventilkolbens eine erste mittlere Öffnung der fünf Öffnungen und eine zur ersten Öffnung benachbarte zweite Öffnung der fünf Öffnungen miteinander über das Ventil gasleitend verbunden sind und eine der zweiten Öffnung bezogen auf die erste Öffnung gegenüberliegende dritte Öffnung der fünf Öffnungen und eine dazu benachbarte randseitige vierte Öffnung der fünf Öffnungen durch das Ventil miteinander gasleitend verbunden sind und die fünf Öffnungen ansonsten im Hohlraum durch das Ventil voneinander gasdicht getrennt sind und in einer zweiten Stellung des Ventilkolbens die erste mittlere Öffnung und die dritte Öffnung miteinander über das Ventil gasleitend verbunden sind und die zweite Öffnung und eine dazu benachbarte randseitige fünfte Öffnung der fünf Öffnungen durch das Ventil miteinander gasleitend verbunden sind und die fünf Öffnungen ansonsten im Hohlraum durch das Ventil voneinander gasdicht getrennt sind.

Hiermit wird eine einfache 2/5-Wege-Ventilsteuerung realisiert, die eine zuverlässige Steuerung des Differenzdruckmotors ermöglicht.

Dabei kann vorgesehen sein, dass der Ventilkolben von der ersten Stellung des Ventilkolbens in die zweite Stellung des Ventilkolbens durch eine Impulsübertragung eines ersten Arbeitskolbens der zwei Arbeitskolben auf den Ventilkolben überführbar ist und der Ventilkolben von der zweiten Stellung des Ventilkolbens in die erste Stellung des Ventilkolbens durch eine Impulsübertragung eines zweiten Arbeitskolbens der zwei Arbeitskolben auf den Ventilkolben überführbar ist.

Hiermit wird erreicht, dass der Ventilkolben auf einfache Weise von den Arbeitskolben anzutreiben ist.

Des Weiteren kann dabei vorgesehen sein, dass der erste Arbeitskolben in der ersten Stellung des Ventilkolbens aufgrund eines auf der Arbeitsfläche des ersten Arbeitskolbens lastenden Drucks in Richtung des Ventilkolbens beschleunigt wird und der zweite Arbeitskolben in der zweiten Stellung des Ventilkolbens aufgrund eines auf der Arbeitsfläche des zweiten Arbeitskolbens lastenden Drucks in Richtung des Ventilkolbens beschleunigt wird.

Hierdurch wird erreicht, dass die Arbeitskolben den nötigen Schwung zur Übertragung eines Impulses auf den Ventilkolben erreichen. Dadurch kann der Ventilkolben aufgrund seiner Trägheit problemlos in beide Stellungen überführt werden, ohne dass der Ventilkolben während des Betriebs des Differenzdruckmotors in einer Totstellung zum Halten kommt.

Ferner kann vorgesehen sein, dass ein erster Arbeitsraum, der durch den ersten Arbeitskolben begrenzt ist, gasleitend mit der zweiten Öffnung verbunden ist und ein zweiter Arbeitsraum, der durch den zweiten Arbeitskolben begrenzt ist, gasleitend mit der dritten Öffnung verbunden ist.

Hierdurch wird eine Steuerung der Druckverhältnisse in den Arbeitsräumen durch eine gemeinsame Nut in der Oberfläche des Ventilkolbens ermöglicht.

Bei bevorzugten Differenzdruckmotoren kann auch vorgesehen sein, dass der Differenzdruckmotor ein Federelement aufweist, das sich an einer geschlossenen Außenseite einer Stirnfläche eines den Hohlraum begrenzenden Gehäuses abstützt und das mit der Stange verbunden ist, so dass ohne eine Krafteinwirkung durch einen Differenzdruck das Federelement die Stange maximal aus dem Hohlraum zieht.

Dadurch wird erreicht, dass der am nächsten zum Federelement angeordnete Arbeitskolben an der Innenseite der Stirnfläche des Hohlraums in der kraftfreien Ruhestellung des Differenzdruckmotors anliegt.

Des Weiteren kann vorgesehen sein, dass die zwei Arbeitskolben einen Durchmesser von größer 10 mm, bevorzugt von größer 20 mm und ganz besonders bevorzugt von größer 30 mm haben.

Hiermit wird eine ausreichende Kraftübertragung für einen mit Vakuum beziehungsweise Unterdruck angetriebenen Differenzdruckmotor ermöglicht, um auch normale medizinische Anwendungen zu ermöglichen.

Es kann vorgesehen sein, dass zumindest eine Antriebsstange an einer äußeren Seite wenigstens eines der zwei Arbeitskolben angeordnet ist, wobei die zumindest eine Antriebsstange durch eine Vorderseite und/oder einer Rückseite eines den Hohlraum begrenzenden Gehäuses hindurch aus dem Gehäuse herausragt und die zumindest eine Antriebsstange in einer Führung in der Vorderseite und/oder der Rückseite des Gehäuses beweglich gelagert ist, wobei vorzugsweise die Stange, die die beiden Arbeitskolben verbindet, durch zumindest einen der Arbeitskolben hindurch geführt ist und dort die zumindest eine Antriebsstange bildet.

Hierdurch wird die Bewegung der Arbeitskolben unmittelbar und auf einfache Weise nutzbar. Bevorzugt ist die zumindest eine Antriebsstange eine Verlängerung der Stange, mit der die Arbeitskolben verbunden sind. Hierzu kann bevorzugt vorgesehen sein, dass die Stange und die zumindest eine Antriebsstange einteilig ausgeführt sind.

Dabei kann vorgesehen sein, dass an der Vorderseite der zumindest einen Antriebsstange ein Befestigungselement, insbesondere ein Gewinde, angeordnet ist, über das ein Werkzeug, wie eine Säge, eine Raspel oder eine Bürste, mit einem zum Befestigungselement passenden Gegenbefestigungselement, insbesondere einem zum Gewinde passenden Gegengewinde, an der zumindest einen Antriebsstange befestigbar ist.

Hierdurch kann ein Werkzeug auf einfache Weise an der zumindest einen Antriebsstange befestigt und auch wieder gelöst werden.

Es kann gemäß einer bevorzugten Weiterbildung auch vorgesehen sein, dass ein den Hohlraum begrenzendes Gehäuse, die zwei Arbeitskolben, der Ventilkolben und die Stange aus einem Kunststoff gefertigt sind, insbesondere aus einem thermoplastischen Kunststoff gefertigt sind, vorzugsweise durch Spritzgießen geformt sind.

Hierdurch kann der Differenzdruckmotor als hygienisches Wegwerf-Produkt in sterilen Umgebungen eingesetzt werden.

Ferner kann vorgesehen sein, dass die geometrische Abmessung des Ventilkolbens in Richtung einer Verbindungslinie zwischen den beiden Arbeitskolben kleiner ist als der Abstand der beiden Arbeitskolben, wobei bevorzugt die geometrische Abmessung des Ventilkolbens in Richtung der Verbindungslinie zwischen den beiden Arbeitskolben maximal 50% kleiner ist als der Abstand zwischen den beiden Arbeitskolben, besonders bevorzugt maximal 10% kleiner ist als der Abstand zwischen den beiden Arbeitskolben.

Hierdurch wird sichergestellt, dass der Ventilkolben zwischen den beiden Arbeitskolben beweglich ist und von den Arbeitskolben angestoßen werden kann, um einen Impulsübertrag auf den Ventilkolben zu generieren und um die Masseträgheit des Ventilkolbens zur Überwindung eines Totpunkts des Ventils zu nutzen.

Des Weiteren kann vorgesehen sein, dass der Differenzdruckmotor eine Starthilfe aufweist, die verhindert, dass der Differenzdruckmotor bei einer Unterbrechung der Zufuhr des Arbeitsmediums in einem Totpunkt anhält, aus dem heraus er nicht mehr von alleine startet, wenn die Zufuhr des Arbeitsmediums in den Differenzdruckmotor wieder erfolgt, wobei vorzugsweise die Starthilfe den Ventilkolben in eine Stellung überführt, in der durch das Arbeitsmedium eine Druckdifferenz zwischen den Arbeitsflächen der Arbeitskolben erzeugbar ist.

Hierdurch wird sichergestellt, dass der Ventilkolben nicht in einer Totstellung verbleibt, die ein erneutes Anlaufen des Differenzdruckmotors verhindert.

Die Auskopplung der Kraft aus dem Differenzdruckmotor kann über die Arbeitskolben oder die Stange beziehungsweise die Antriebsstange(n) erfolgen. Alternativ kann auch eine Kopplung über Magnetfelder erfolgen, beispielsweise, indem einer oder beide der Arbeitskolben oder die Antriebsstange oder beide Antriebsstangen magnetisch sind. Mit dem sich bewegenden Magnetfeld kann dann ein über das Magnetfeld gekoppeltes Antriebsteil und damit ein Werkzeug außerhalb des Differenzdruckmotors angetrieben werden. Ferner kann auch ein Stromfluss in einer Spule angeregt werden.

Die der vorliegenden Erfindung zugrundeliegenden Aufgaben werden auch gelöst durch ein chirurgisches Antriebssystem aufweisend einen erfindungsgemäßen Differenzdruckmotor sowie ein Ventilelement, insbesondere ein manuell bedienbares Ventilelement, wobei das Ventilelement in einer Leitung angeordnet ist, die mit einer der fünf Öffnungen oder mit zwei der fünf Öffnungen verbunden ist und die mit einer Unterdruckquelle oder einem Druckgasreservoir oder einer Pumpe verbindbar oder verbunden ist, so dass mit dem Ventilelement die Verbindung zur Unterdruckquelle, dem Druckgasreservoir oder der Pumpe unterbrechbar und/oder der Druck an der einen Öffnung der fünf Öffnungen oder der zwei Öffnungen der fünf Öffnungen einstellbar ist.

Das chirurgische Antriebssystem weist dann die gleichen Vorteile auf, wie der Differenzdruckmotor.

Es kann vorgesehen sein, dass das Antriebssystem ein Gehäuse mit mindestens eine gasdurchlässige Durchführung aufweist, die den Innenraum des Gehäuses mit der umgebenden Atmosphäre verbindet.

Ferner kann vorgesehen sein, dass ein Sterilfilter zwischen dem Ventil und der Unterdruckquelle angeordnet ist.

Es kann vorgesehen sein, dass das chirurgische Antriebssystem einen Griff aufweist, mit dem es in einer Hand haltbar ist und das Ventilelement mit einem Abzug am Griff bedienbar ist.

Hierdurch ist das chirurgische Antriebssystem auf einfache Weise mit einer Hand einsetzbar.

Es kann auch vorgesehen sein, dass das chirurgische Antriebssystem ein äußeres Gehäuse aufweist, das ein den Hohlraum des Differenzdruckmotors begrenzendes Gehäuse umschließt und das eine Öffnung oder eine Führung aufweist, durch die die Stange oder eine Antriebsstange aus dem äußeren Gehäuse herausragt.

Die der vorliegenden Erfindung zugrundeliegenden Aufgaben werden ferner gelöst durch ein medizinisches Lavage-System zum Debridement von Weichgewebe und/oder Knochengewebe aufweisend einen erfindungsgemäßen Differenzdruckmotor oder ein erfindungsgemäßes chirurgisches Antriebssystem oder eine medizinische Vorrichtung zum Bürsten, Raspeln oder Sägen von Weichgewebe und/oder Knochengewebe aufweisend einen erfindungsgemäßen Differenzdruckmotor oder ein erfindungsgemäßes chirurgisches Antriebssystem.

Diese Systeme sind im medizinischen Bereich, insbesondere im OP-Bereich, besonders sinnvoll mit dem Differenzdruckmotor anzutreiben.

Die der vorliegenden Erfindung zugrundeliegenden Aufgaben werden des Weiteren gelöst durch ein Verfahren zum Betreiben eines Differenzdruckmotors, bei dem ein erster Arbeitskolben und ein zweiter Arbeitskolben über eine Stange verbunden sind und linear in einem Hohlraum oszillieren, wobei das Verfahren die folgenden Schritte umfasst:
A) ein Ventilkolben, der zwischen dem ersten Arbeitskolben und dem zweiten Arbeitskolben im Hohlraum angeordnet ist, befindet sich in einer ersten Stellung;
B) Bereitstellen einer Verbindung zwischen einem Vakuumanschluss zu einem ersten Arbeitsraum und einer Verbindung zwischen einem zweiten Arbeitsraum und der Umgebung des Differenzdruckmotors in der ersten Stellung des Ventilkolbens durch den Ventilkolben, wobei der erste Arbeitsraum von dem ersten Arbeitskolben begrenzt ist und der zweite Arbeitsraum von dem zweiten Arbeitskolben begrenzt ist;
C) Evakuieren von Gas aus dem ersten Arbeitsraum und dadurch Bewegen des ersten Arbeitskolbens und des zweiten Arbeitskolbens in dem Hohlraum;
D) Anstoßen des Ventilkolbens mit dem zweiten Arbeitskolben, so dass der Ventilkolben in eine zweite Stellung überführt wird;
E) Bereitstellen einer Verbindung zwischen dem Vakuumanschluss zu dem zweiten Arbeitsraum und einer Verbindung zwischen dem ersten Arbeitsraum und der Umgebung des Differenzdruckmotors in der zweiten Stellung des Ventilkolbens durch den Ventilkolben;
F) Evakuieren von Gas aus dem zweiten Arbeitsraum und Einströmen von Umgebungsluft in den ersten Arbeitsraum und dadurch umgekehrtes Bewegen des ersten Arbeitskolbens und des zweiten Arbeitskolbens in dem Hohlraum;
G) Anstoßen des Ventilkolbens mit dem ersten Arbeitskolben, so dass der Ventilkolben in die erste Stellung überführt wird.

Die der vorliegenden Erfindung zugrundeliegenden Aufgaben werden des Weiteren auch gelöst Verfahren zum Betreiben eines Differenzdruckmotors, bei dem ein erster Arbeitskolben und ein zweiter Arbeitskolben über eine Stange verbunden sind und linear in einem Hohlraum oszillieren, wobei das Verfahren die folgenden Schritte umfasst:
A) ein Ventilkolben, der zwischen dem ersten Arbeitskolben und dem zweiten Arbeitskolben im Hohlraum angeordnet ist, befindet sich in einer ersten Stellung;
B) Bereitstellen einer Verbindung zwischen einem Druckgasanschluss zu einem ersten Arbeitsraum und einer Verbindung zwischen einem zweiten Arbeitsraum und der Umgebung des Differenzdruckmotors in der ersten Stellung des Ventilkolbens durch den Ventilkolben, wobei der erste Arbeitsraum von dem ersten Arbeitskolben begrenzt ist und der zweite Arbeitsraum von dem zweiten Arbeitskolben begrenzt ist;
C) Erhöhen des Gasdrucks in dem ersten Arbeitsraum und dadurch Bewegen des ersten Arbeitskolbens und des zweiten Arbeitskolbens in dem Hohlraum;
D) Anstoßen des Ventilkolbens mit dem ersten Arbeitskolben, so dass der Ventilkolben in eine zweite Stellung überführt wird;
E) Bereitstellen einer Verbindung zwischen dem Druckgasanschluss zu dem zweiten Arbeitsraum und einer Verbindung zwischen dem ersten Arbeitsraum und der Umgebung des Differenzdruckmotors in der zweiten Stellung des Ventilkolbens durch den Ventilkolben;
F) Erhöhen des Gasdrucks in dem zweiten Arbeitsraum und Ausströmen von Druckgas aus dem ersten Arbeitsraum und dadurch umgekehrtes Bewegen des ersten Arbeitskolbens und des zweiten Arbeitskolbens in dem Hohlraum;
G) Anstoßen des Ventilkolbens mit dem zweiten Arbeitskolben, so dass der Ventilkolben in die erste Stellung überführt wird.

Dabei kann vorgesehen sein, dass das Verfahren mit einem erfindungsgemäßen Differenzdruckmotor oder mit einem erfindungsgemäßen chirurgischen Antriebssystem oder mit einem erfindungsgemäßen Lavage-System oder einer erfindungsgemäßen medizinischen Vorrichtung zum Bürsten, Raspeln oder Sägen von Weichgewebe und/oder Knochengewebe durchgeführt wird.

Ferner kann vorgesehen sein, dass der Vakuumanschluss oder der Druckgasanschluss durch eine erste mittlere Öffnung in einen Ventilraum münden, in dem sich der Ventilkolben bewegt, wobei neben der ersten mittleren Öffnung eine zweite Öffnung angeordnet ist, die den Ventilraum mit dem ersten Arbeitsraum verbindet, neben der ersten mittleren Öffnung eine dritte Öffnung angeordnet ist, die den Ventilraum mit dem zweiten Arbeitsraum verbindet, neben der zweiten Öffnung eine vierte äußere Öffnung angeordnet ist, die den Ventilraum mit der Umgebung des Differenzdruckmotors verbindet, und neben der dritten Öffnung eine fünfte äußere Öffnung angeordnet ist, die den Ventilraum mit der Umgebung des Differenzdruckmotors verbindet, wobei in dem Ventilkolben drei Verbindungen, insbesondere drei Nuten, angeordnet sind, so dass in der ersten Stellung des Ventilkolbens die mittlere erste Öffnung mit der zweiten Öffnung gasdurchlässig verbunden wird und die dritte Öffnung mit der fünften Öffnung gasdurchlässig verbunden wird und in der zweiten Stellung des Ventilkolbens die mittlere erste Öffnung mit der dritten Öffnung gasdurchlässig verbunden wird und die zweite Öffnung mit der vierten Öffnung gasdurchlässig verbunden wird, wobei vorzugsweise die fünf Öffnungen ansonsten voneinander durch den Ventilkolben getrennt werden.

Hierdurch kann der Differenzdruckmotor besonders einfach und kostengünstig aufgebaut werden und das Verfahren kann zuverlässig ablaufen.

Des Weiteren kann vorgesehen sein, dass nach Schritt G) eine Wiederholung der Schritte B) bis G) erfolgt, solange am Vakuumanschluss ein Vakuum oder ein Unterdruck anliegt oder solange am Druckgasanschluss ein Überdruck anliegt oder ein Druckgas eingespeist wird.

Hierdurch kann das Verfahren am Laufen gehalten werden, solange ein ausreichender Unterdruck oder ein ausreichender Gasdruck vorhanden ist. Zusätzlich kann der Differenzdruckmotor natürlich auch durch ein Blockieren der Arbeitskolben angehalten werden, beispielsweise durch einen hohen Widerstand gegen die Bewegung.

Bevorzugt kann auch vorgesehen sein, dass mit der Bewegung der Arbeitskolben ein Werkzeug oder eine Pumpe angetrieben wird, insbesondere ein medizinisches Werkzeug, wie eine Säge, eine Raspel oder eine Bürste, oder ein Lavage-System angetrieben wird.

Hierdurch wird die Bewegung der Arbeitskolben des Differenzdruckmotors für den Anwender nutzbar.

Ferner wird mit der vorliegenden Erfindung vorgeschlagen, dass der Differenzdruckmotor durch Öffnen eines Ventilelements in einer Vakuumleitung am Vakuumanschluss oder in einer Druckgasleitung am Druckgasanschluss angeschaltet und durch Schließen des Ventilelements ausgeschaltet wird.

Hiermit kann die Bewegung der Arbeitskolben und damit der Differenzdruckmotor auf einfache Weise eingestellt werden.

Der Erfindung liegt die überraschende Erkenntnis zugrunde, dass es durch die Anordnung eines Ventilkolbens zwischen zwei Arbeitskolben gelingt, einen einfachen Differenzdruckmotor bereitzustellen, der kostengünstig aus Kunststoff zu fertigen ist und der gleichzeitig zuverlässig läuft. Der Ventilkolben ist dabei Teil eines Ventils, mit dem eine alternierende Beaufschlagung der Arbeitskolben mit unterschiedlichen Drucken (dem ersten Druck und dem zweiten Druck) gesteuert wird. Der Ventilkolben kann durch seine Anordnung zwischen den Arbeitskolben von den Arbeitskolben angetrieben werden. Hierzu ist es besonders vorteilhaft, wenn der von den Arbeitskolben auf den Ventilkolben übertragene Impuls dazu ausreicht und die Trägheit des Ventilkolbens dazu ausreicht, dass der Ventilkolben über eine Totpunkt-Stellung hinausläuft, in der keiner der Arbeitskolben mit einem Druckgas oder einem Vakuum beziehungsweise einem Unterdruck beaufschlagt wird. Durch den erzeugten Hub erzielen die Arbeitskolben eine kraftvolle Bewegung eines damit verbundenen Werkzeugs, der für verschiedene Anwendungen im OP-Bereich einsetzbar ist.

Ein beispielhafter erfindungsgemäßer Differenzdruckmotor ist zusammengesetzt aus
a) ein erster Arbeitskolben und ein zweiter Arbeitskolben als die zwei Arbeitskolben, die mit einer Stange verbunden sind, wobei die Arbeitskolben mit der Stange axial verschiebbar in einem zumindest bereichsweise zylindrischen Hohlraum angeordnet sind,
b) einem Ventilkolben, der zwischen den beiden Arbeitskolben auf der Stange frei axial verschiebbar angeordnet ist, wobei der Ventilkolben zusammen mit Öffnungen im Hohlraum ein 2/5-Wegeventil bildet,
c) einem ersten Arbeitsraum als Teil des Hohlraums, in dem der erste Arbeitskolben bewegt werden kann,
d) einem zweiten Arbeitsraum als Teil des Hohlraums, in dem der zweite Arbeitskolben bewegt werden kann,
e) Wobei das 2/5-Wegeventil mit einer Vakuumzuleitung, mit der umgebenden Atmosphäre, mit dem ersten Arbeitsraum und dem zweiten Arbeitsraum verbunden ist, und wobei
f) bei Beaufschlagung mit Vakuum die beiden Arbeitskolben durch periodische Wechsel der Einwirkung von Vakuum und der umgebenden Atmosphäre eine oszillierende Bewegung der Stange bewirken, wobei die Arbeitskolben periodisch den Ventilkolben anstoßen und eine axiale Bewegung des Ventilkolbens verursachen, wodurch ein Umschalten des Ventils bewirkt wird.

Ein weiterer beispielhafter erfindungsgemäßer Differenzdruckmotor ist zusammengesetzt aus
a) einem zumindest bereichsweise zylindrischen Hohlraum, der durch zwei geschlossene Stirnflächen begrenzt wird,
b) einer Stange, der im Hohlraum axial beweglich angeordnet ist, wobei die Stange durch Aussparungen in den Stirnflächen aus dem Hohlraum herausragt,
c) einem ersten Arbeitskolben, der mit der Stange nicht verschiebbar verbunden ist,
d) einem ersten Arbeitsraum, der durch den eine Vorderseite (Arbeitsfläche) des ersten Arbeitskolbens, einer zylindrischen Innenwand des Hohlraums und einer Stirnfläche des Hohlraums begrenzt ist,
e) einem zweiten Arbeitskolben, der mit der Stange nicht verschiebbar verbunden ist,
f) einem zweiten Arbeitsraum, der durch den eine Rückseite (Arbeitsfläche) des zweiten Arbeitskolbens, einer zylindrischen Innenwand des Hohlraums und einer Stirnfläche des Hohlraums begrenzt ist,
g) einem Ventilraum, der von der Rückseite des ersten Arbeitskolbens, der Vorderseite des zweiten Arbeitskolbens und einer zumindest bereichsweise zylindrischen Innenwand des Hohlraums begrenzt wird, wobei der Hohlraum im Bereich des Ventilraums fünf Öffnungen eines 2/5-Wegeventil aufweist, die mit dem Ventilraum gasdurchlässig verbunden sind,
h) einem Ventilkolben, der axial frei beweglich auf der Stange im Ventilraum zwischen dem ersten Arbeitskolben und dem zweiten Arbeitskolben angeordnet ist, wobei der Ventilkolben drei umlaufende Nuten besitzt, die axial nebeneinander angeordnet sind, so dass diese mit den fünf Öffnungen des 2/5-Wegeventils wechselwirken können,
i) einer ersten Öffnung der fünf Öffnungen, die an eine Vakuumzuleitung oder eine Druckgasleitung angeschlossen ist, die mit dem 2/5-Wegeventil gasdurchlässig verbunden ist,
j) einer zweiten Öffnung der fünf Öffnungen in einer zylindrischen Wandung des Hohlraums oder in der ersten Stirnfläche, die mit dem 2/5-Wegeventil und dem ersten Arbeitsraum verbunden ist,
k) einer dritten Öffnung der fünf Öffnungen in einer zylindrischen Wandung des Hohlraums oder in der zweiten Stirnfläche, die mit dem 2/5-Wegeventil und dem zweiten Arbeitsraum verbunden ist,
l) zwei weiteren Öffnungen der fünf Öffnungen in einer zylindrischen Wandung des Hohlraums, die mit der Umgebungsatmosphäre und dem 2/5-Wegeventil verbunden sind,
m) wobei der axiale Abstand einer ersten Stirnfläche des Ventilkolbens zur Rückseite des ersten Arbeitskolbens kleiner oder gleich ist, als der Abstand der Vorderseite des ersten Arbeitskolbens zur Innenseite der ersten Stirnfläche des Hohlraums, und
n) wobei der axiale Abstand einer zweiten Stirnfläche des Ventilkolbens zur Vorderseite des zweiten Arbeitskolbens kleiner oder gleich ist, als der Abstand der Rückseite des zweiten Arbeitskolbens zur Innenseite der zweiten Stirnfläche des Hohlraums.

Der erfindungsgemäße Differenzdruckmotor wird vorzugsweise als Antrieb von Lavage-Systemen und von Vorrichtungen zum Debridement von Weichgewebe und Knochengewebe verwendet. Der Differenzdruckmotor wird als Antrieb von Vorrichtungen zum Bürsten, Raspeln und Sägen von Weichgewebe und Knochengewebe verwendet. Weiterhin wird der Differenzdruckmotor zum Antrieb von einmalig zu verwendenden medizinischen Vorrichtungen verwendet.

Im Folgenden werden weitere Ausführungsbeispiele der Erfindung anhand von elf schematisch dargestellten Figuren erläutert, ohne jedoch dabei die Erfindung zu beschränken. Dabei zeigt:
Figur 1: eine schematische Querschnittansicht eines ersten beispielhaften erfindungsgemäßen Differenzdruckmotors im Ruhezustand;
Figur 2: eine schematische Querschnittansicht des Differenzdruckmotors nach Figur 1 mit angelegtem Vakuum;
Figur 3: eine schematische Querschnittansicht des Differenzdruckmotors nach den Figuren 1 und 2 beim Auftreffen des zweiten Arbeitskolbens auf den Ventilkolben;
Figur 4: eine schematische Querschnittansicht des Differenzdruckmotors nach den Figuren 1 bis 3 während des Betriebs, wobei der Ventilkolben in eine erste Stellung überführt ist;
Figur 5: eine schematische Querschnittansicht des Differenzdruckmotors nach den Figuren 1 bis 4 während des Betriebs, wobei der Ventilkolben in eine zweite Stellung überführt ist;
Figur 6: eine schematische Querschnittansicht eines ersten erfindungsgemäßen chirurgischen Antriebssystems aufweisend einen zweiten beispielhaften erfindungsgemäßen Differenzdruckmotor mit Starthilfe;
Figur 7: eine schematische Querschnittansicht einer erfindungsgemäßen medizinischen Vorrichtung zum Bürsten, Raspeln oder Sägen von Weichgewebe und/oder Knochengewebe;
Figur 8: eine schematische Querschnittansicht eines dritten beispielhaften erfindungsgemäßen Differenzdruckmotors während des Betriebs mit angelegtem Druckgas;
Figur 9: eine schematische Querschnittansicht eines zweiten erfindungsgemäßen chirurgischen Antriebssystems aufweisend einen vierten beispielhaften erfindungsgemäßen Differenzdruckmotor mit Starthilfe im Ausgangszustand;
Figur 10: eine schematische Querschnittansicht des Differenzdruckmotors nach Figur 9 während des Betriebs, wobei der Ventilkolben in eine erste Stellung überführt ist; und
Figur 11: eine schematische Querschnittansicht des Differenzdruckmotors nach Figur 9 und 10 während des Betriebs, wobei der Ventilkolben in eine zweite Stellung überführt ist.

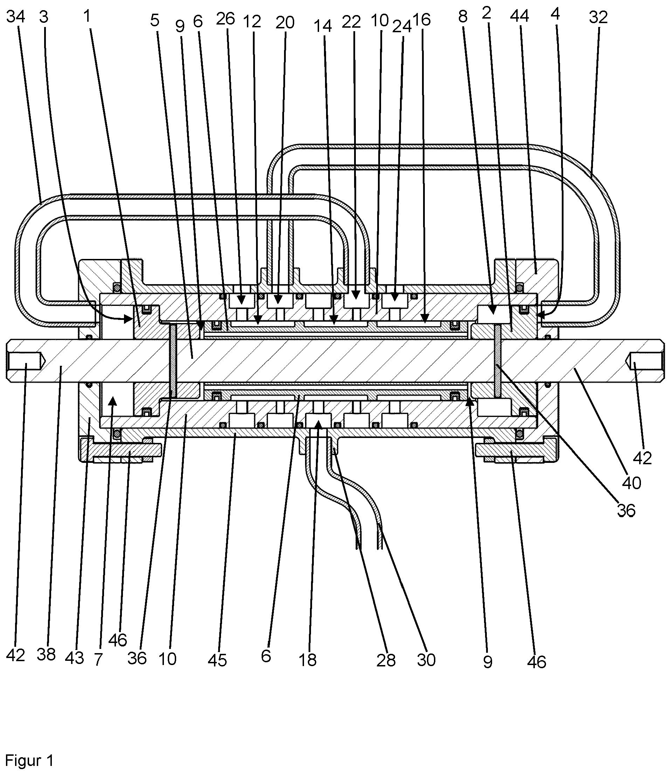

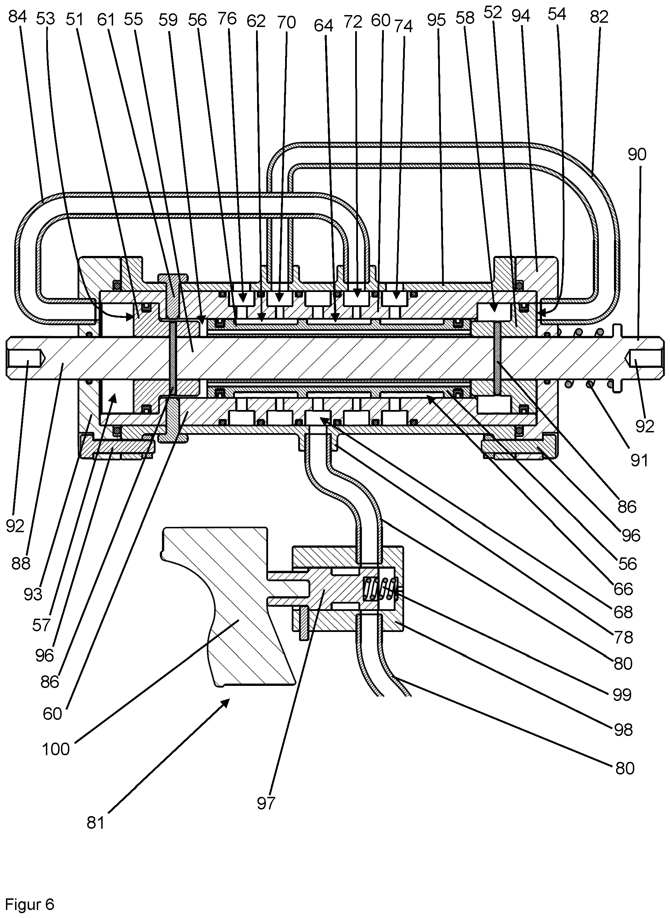

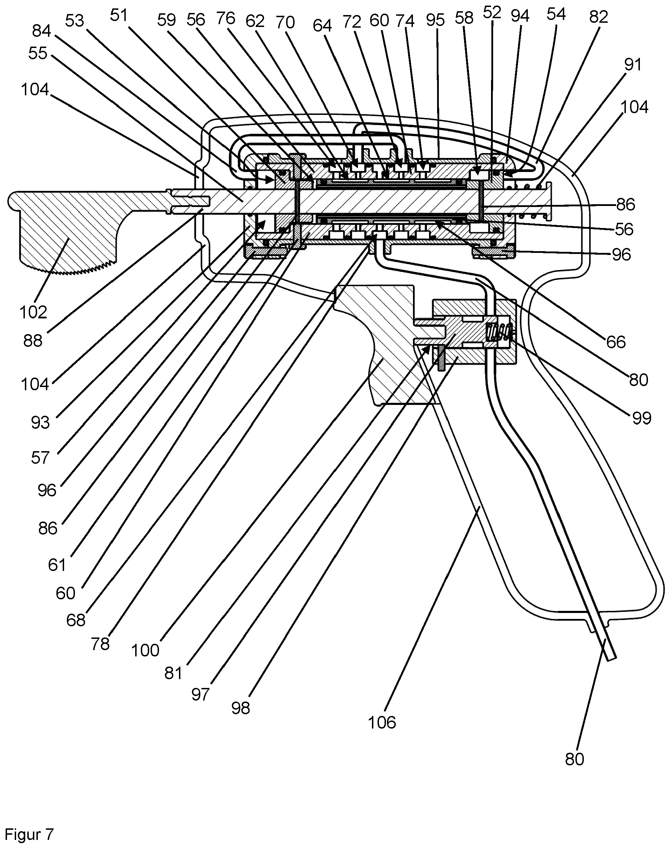

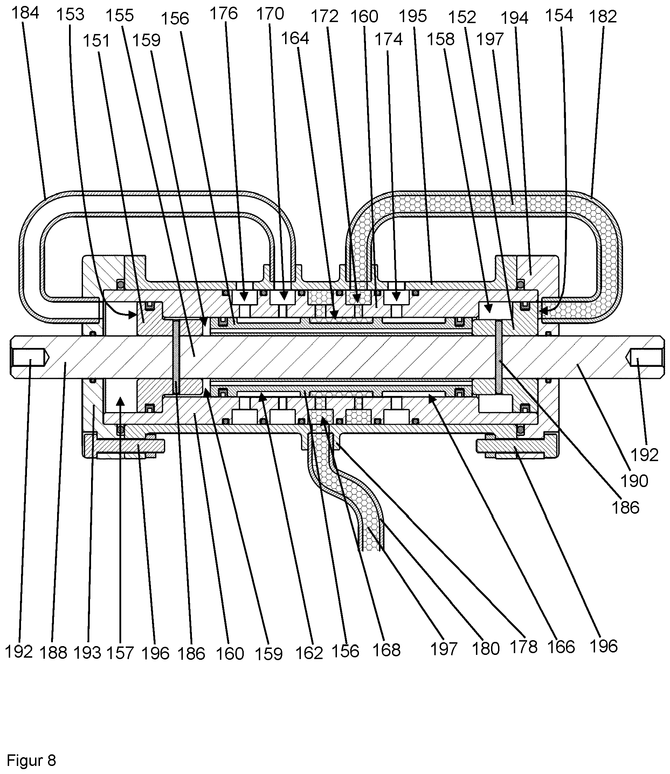

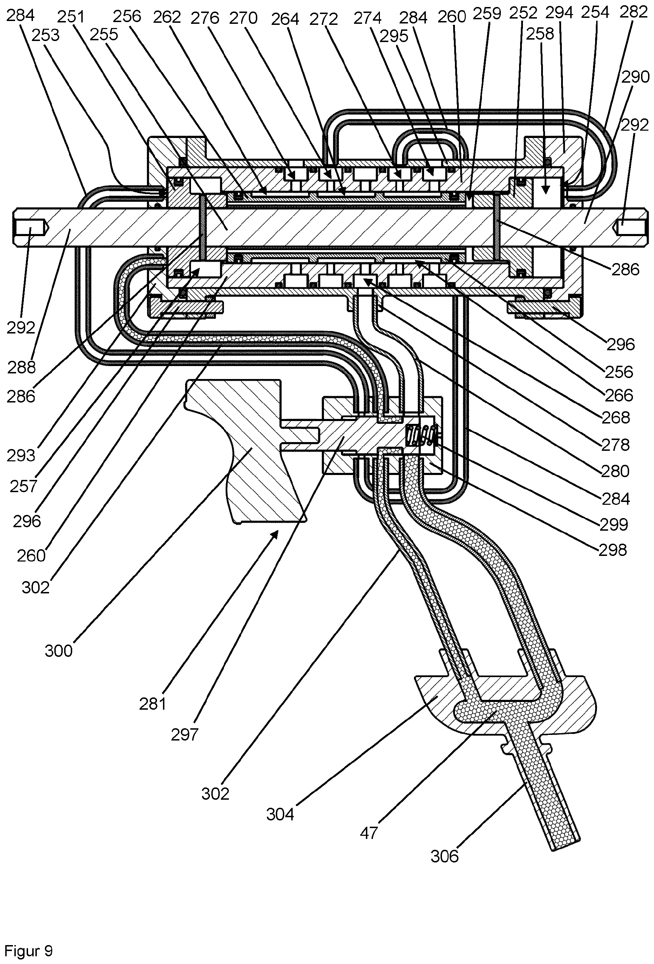

In den Figuren 1 bis 5 sind Querschnittansichten eines ersten beispielhaften erfindungsgemäßen Differenzdruckmotors gezeigt, mit denen der Ablauf eines erfindungsgemäßen Verfahrens verdeutlicht wird. Die Figuren 6 und 7 zeigen ein erfindungsgemäßes chirurgisches Antriebssystem und eine erfindungsgemäße medizinischen Vorrichtung mit einem zweiten erfindungsgemäßen Differenzdruckmotor. Der erste beispielhafte Differenzdruckmotor nach den Figuren 1 bis 5 könnte aber auch ohne weiteres auch in dem chirurgisches Antriebssystem nach Figur 6 und in der medizinischen Vorrichtung nach Figur 7 eingesetzt werden. In Figur 8 ist ein dritter beispielhafter erfindungsgemäßer Differenzdruckmotor gezeigt, der mit einem Druckgas angetrieben werden kann. Die Figuren 9 bis 11 zeigen ein zweites erfindungsgemäßes chirurgisches Antriebssystem mit einem vierten beispielhaften erfindungsgemäßen Differenzdruckmotor.

Die Vorderseiten der Differenzdruckmotoren, der chirurgischen Antriebssysteme und der medizinischen Vorrichtung sind in allen Figuren links, wobei die beidseitig wirkenden Differenzdruckmotoren nach den Figuren 1 bis 6 und 8 bis 11 neben einem linear oszillierenden Antrieb an der Vorderseite grundsätzlich auch dazu vorgesehen sein können, ein Werkzeug oder eine Pumpe mit der Rückseite anzutreiben.

Der erste beispielhafte erfindungsgemäße Differenzdruckmotor weist einen ersten Arbeitskolben 1 und einen zweiten Arbeitskolben 2 auf. Der erste Arbeitskolben 1 kann eine in Richtung der Vorderseite des Differenzdruckmotors (in den Figuren 1 bis 5 links) weisende Arbeitsfläche 3 aufweisen. Analog dazu kann der zweite Arbeitskolben 2 eine in Richtung der Rückseite des Differenzdruckmotors (in den Figuren 1 bis 5 rechts) weisende Arbeitsfläche 4 aufweisen. Die Arbeit an den beiden Arbeitskolben 1, 2 kann so auf jeweils gegenüberliegenden Seiten geleistet werden, so dass der Druckgasmotor beidseitig angetrieben werden kann. Der erste Arbeitskolben 1 und der zweite Arbeitskolben 2 können über eine Stange 5 fest miteinander verbunden sein. Die Stange 5 kann die beiden Arbeitskolben 1, 2 in einem festen Abstand zueinander halten. Ferner kann durch die Stange 5 die Ausrichtung der Arbeitskolben 1, 2 zueinander fixiert sein.

Zwischen den beiden Arbeitskolben 1, 2 kann um die Stange 5 herum ein hülsenförmiger Ventilkolben 6 angeordnet sein, der auf der Stange 5 axial (bezogen auf die Stange 5) beweglich gelagert ist. Der Ventilkolben 6 kann in seiner axialen Ausdehnung kleiner sein, als der durch die Stange 5 bestimmte Abstand der beiden Arbeitskolben 1, 2. Hierdurch kann der Ventilkolben 6 zwischen den beiden Arbeitskolben 1, 2 beweglich sein. Erfindungsgemäß bevorzugt kann vorgesehen sein, dass der Ventilkolben 6 bezogen auf die Länge der Stange 5 zwischen den beiden Arbeitskolben 1, 2, die den Abstand zwischen den beiden Arbeitskolben 1, 2 bestimmt, kleiner ist als der Abstand zwischen den beiden Arbeitskolben 1, 2. Hierdurch wird eine Impulsübertragung zwischen den von den Arbeitsflächen 3, 4 abgewandten Seiten der Arbeitskolben 1, 2 auf den Ventilkolben 6 mittels eines Stoßes möglich und die Massenträgheit des Ventilkolbens 6 kann dazu genutzt werden, den Ventilkolben 6 über einen Totpunkt eines mit dem Ventilkolbens 6 aufgebauten Ventils hinweg zu stoßen.

Die Arbeitskolben 1, 2, die Stange 5 und der Ventilkolben 6 können sich in einem bereichsweise zylindrischen Hohlraum befinden. Der erste Arbeitskolben 1 kann in einem ersten zylindrischen Arbeitsraum 7 angeordnet sein. Der zweite Arbeitskolben 2 kann in einem zweiten zylindrischen Arbeitsraum 8 angeordnet sein. Der Ventilkolben 6 kann in einem zylindrischen Ventilraum 9 angeordnet sein. Der erste zylindrische Arbeitsraum 7 und der zweite zylindrische Arbeitsraum 8 können einen größeren Durchmesser haben als der dazwischen angeordnete Ventilraum 9. Die Arbeitskolben 1, 2 können in den Arbeitsräumen 7, 8 in axialer Richtung beweglich angeordnet sein. Die Arbeitskolben 1, 2 können auf der der jeweiligen Arbeitsfläche 3, 4 gegenüberliegenden Seite einen Fortsatz mit geringerem Durchmesser aufweisen, der in den Ventilraum 9 hineinreicht. Dadurch können die Arbeitskolben 1, 2 mit ihren Fortsätzen im Ventilraum 9 auf den Ventilkolben 6 stoßen. Die Arbeitskolben 1, 2 können im Bereich der Arbeitsflächen 3, 4 einen zum Innenumfang des jeweiligen Arbeitsraums 7, 8 passenden Außenumfang aufweisen. Bevorzugt dichten die Arbeitskolben den jeweiligen Arbeitsraum 7, 8 gasdicht oder druckdicht ab. Hierzu können am Außenumfang der Arbeitskolben 1, 2 umlaufende Kolbenringe (siehe Figuren 1 bis 5) oder andere Dichtungen vorgesehen sein.

Der Ventilkolben 6 kann außen eine zum zylindrischen Ventilraum 9 passende zylindrische Form aufweisen. Der Ventilkolben 6 kann am Außenumfang an seinen beiden den Arbeitskolben 1, 2 zugewandten Enden je einen umlaufenden Kolbenring (siehe Figuren 1 bis 5) oder anderen Dichtungsring aufweisen. Im Inneren des Ventilkolbens 6 kann ein gasdurchlässiger Durchgang angeordnet sein, der die beiden den Arbeitskolben 1, 2 zugewandten Seiten des Ventilkolbens 6 gasdurchlässig miteinander verbindet.

Die beiden Arbeitsräume 7, 8 und der Ventilraum 9 bilden zusammen den bereichsweise zylindrischen Hohlraum, in dem die Arbeitskolben 1, 2 mit der Stange 5 und dem Ventilkolben 6 beweglich angeordnet sind. Der Hohlraum kann durch einen Hohlkörper aus Kunststoff geformt werden. Der Hohlraum kann durch Wandungen 10 des Hohlraums begrenzt sein.

In einem ansonsten zylindrischen Außenumfang des Ventilkolbens 6 können drei umlaufende rotationssymmetrische Nuten 12, 14, 16 angeordnet sein. Diese Nuten 12, 14, 16 können mit der sie umgebenden Wandung 10 drei voneinander getrennte ringförmige Hohlräume bilden. Diese können zum Schalten eines Ventils verwendet werden, das mit dem Ventilkolben 6 gebildet wird. Zur Bildung eines solchen Ventils können im Bereich des Ventilraums 9 fünf durchgehende Öffnungen 18, 20, 22, 24, 26 in der Wandung 10 angeordnet sein. Die fünf durchgehenden Öffnungen 18, 20, 22, 24, 26 können axial (bezogen auf den zylindrischen Ventilraum 9) nebeneinander angeordnet sein. Eine erste mittlere Öffnung 18 kann in einen Vakuumanschluss 28 münden. Eine Vakuumleitung 30 in Form eines Schlauchs kann an den Vakuumanschluss 28 angeschlossen sein. Die Vakuumleitung 30 kann den Vakuumanschluss 28 und damit die erste Öffnung 18 mit einer Vakuumquelle oder Unterdruckquelle verbinden. Eine zweite Öffnung 20, die axial neben der ersten Öffnung 18 angeordnet sein kann, kann über eine Leitung 32 gasdurchlässig mit dem zweiten Arbeitsraum 8 verbunden sein. Eine dritte Öffnung 22, die axial neben der ersten Öffnung 18 aber gegenüberliegend der zweiten Öffnung 20 angeordnet sein kann, kann über eine Leitung 34 gasdurchlässig mit dem ersten Arbeitsraum 7 verbunden sein. Eine vierte außen liegende Öffnung 24 kann gasdurchlässig mit der Umgebung des Differenzdruckmotors verbunden sein. Eine fünfte außen liegende Öffnung 26 kann gasdurchlässig mit der Umgebung des Differenzdruckmotors verbunden sein.

Die axial nebeneinanderliegenden durchgehenden Öffnungen 18, 20, 22, 24, 26 können mit der Wandung 10 und dem axial innerhalb des Ventilraums 9 beweglichen und gegen die Wandung 10 abgedichteten Ventilkolben 6 und den Nuten 12, 14, 16 das Ventil bilden, mit dem der Differenzdruckmotor gesteuert wird. Die Bewegung des Ventilkolbens 6 kann dabei durch die Arbeitskolben 1, 2 angeregt werden, die von beiden Seiten auf den Ventilkolben 6 stoßen und so zu einer Oszillation anregen können. Die Nuten 12, 14, 16 können bevorzugt so breit sein, dass jeweils zwei benachbarte Öffnungen 18, 20, 22, 24, 26 gleichzeitig in eine der Nuten 12, 14, 16 münden. Hierdurch können je nach Stellung des Ventilkolbens 6 immer zwei Öffnungen 18, 20, 22, 24, 26 miteinander gasdurchlässig verbunden sein. Die Nuten 12, 14, 16 sind bevorzugt aber in axialer Richtung nicht so breit, dass drei Öffnungen 18, 20, 22, 24, 26 in die gleiche Nut 12, 14, 16 münden können. Ferner kann vorzugsweise die Wand zwischen den Nuten 12, 14, 16 in axialer Richtung so breit sein, dass sie zumindest die zweite Öffnung 20 und die dritte Öffnung 22 geradeso abdecken kann, so dass ausgeschlossen ist, dass die zweite Öffnung 20 oder die dritte Öffnung 22 gleichzeitig in zwei der Nuten 12, 14, 16 münden kann und so einen "Kurzschluss" des Differenzdruckmotors bewirkt. Aus diesem Grund ist es auch wichtig, dass der Ventilkolben 6, insbesondere aufgrund seiner Trägheit, automatisch den Punkt überfährt, bei dem die zweite Öffnung 20 oder die dritte Öffnung 22 geschlossen sind und in keine der Nuten 12, 14, 16 münden. Hierdurch wird eine Totstellung des Differenzdruckmotors vermieden.

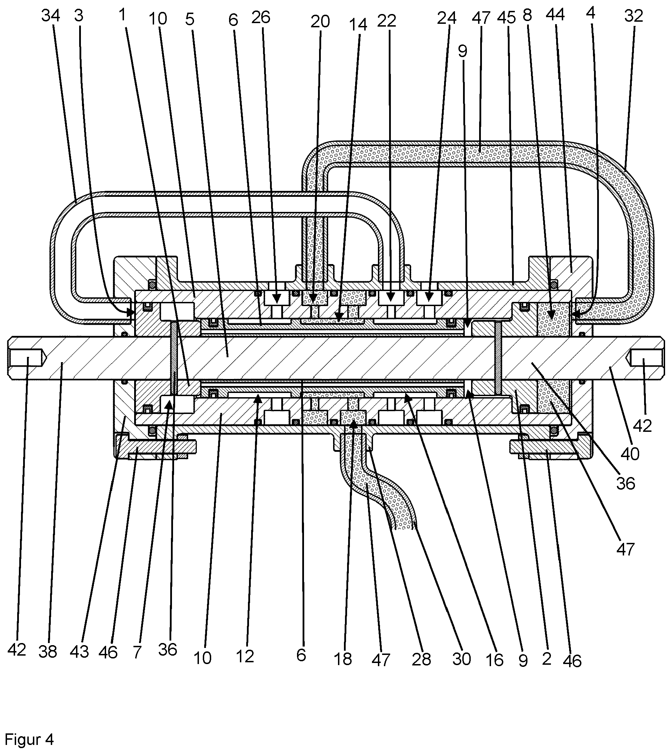

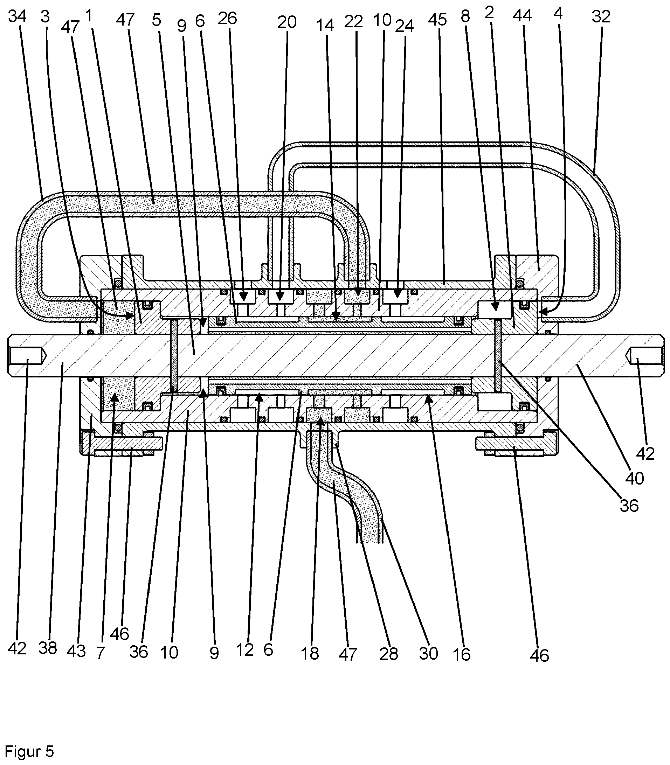

In einer ersten Stellung des Ventilkolbens 6 (siehe Figur 4) kann die erste mittlere Öffnung 18 und die benachbarte zweite Öffnung 20 miteinander über die mittlere Nut 14 gasleitend verbunden sein und die dritte Öffnung 22 und die dazu benachbarte randseitige vierte Öffnung 24 durch die rückseitige Nut 16 miteinander gasleitend verbunden sein und die fünf Öffnungen 18, 20, 22, 24, 26 ansonsten durch das Ventil voneinander gasdicht getrennt sein. In einer zweiten Stellung des Ventilkolbens 6 (siehe Figur 5) kann die erste mittlere Öffnung 18 und die dritte Öffnung 22 miteinander über die mittlere Nut 14 gasleitend verbunden sein und die zweite Öffnung 20 und die dazu benachbarte randseitige fünfte Öffnung 26 durch die vorderseitige Nut 12 miteinander gasleitend verbunden sein und die fünf Öffnungen 18, 20, 22, 24, 26 ansonsten durch das Ventil voneinander gasdicht getrennt sein. Der Ventilkolben 6 kann durch die Arbeitskolben 1, 2 von der ersten Stellung in die zweite Stellung überführt werden. Bei Einwirken eines Vakuums 47 am Vakuumanschluss 28 kann in der ersten Stellung des Ventilkolbens 6 der zweite Arbeitsraum 8 evakuiert werden. Gleichzeitig kann der erste Arbeitsraum 7 über die dritte Öffnung 22, die vierte Öffnung 24 und die hintere Nut 16 mit der Umgebung gasdurchlässig verbunden sein, so dass Luft in den ersten Arbeitsraum 7 strömt beziehungsweise vorhanden ist. Durch diese Druckdifferenz kann der erste Arbeitskolben 1 nach hinten gedrückt werden, beziehungsweise der zweite Arbeitskolben 2 nach hinten gezogen werden (in den Figuren 1 bis 5 nach rechts). Wenn der erste Arbeitskolben 1, der mit dem zweiten Arbeitskolben 2 über die Stange 5 verbunden ist, nach hinten gezogen wird und trifft dieser auf den Ventilkolben 6. Der Ventilkolben 6 kann dadurch aus der ersten Stellung herausgestoßen werden und in die zweite Stellung überführt werden. In dieser zweiten Stellung des Ventilkolbens 6 (siehe Figur 5) kann der erste Arbeitsraum 7 evakuiert werden, während er von der Umgebung getrennt ist. Gleichzeitig kann der zweite Arbeitsraum 8 über die zweite Öffnung 20, die fünfte Öffnung 26 und die vordere Nut 12 mit der Umgebung gasdurchlässig verbunden sein, so dass Luft in den zweiten Arbeitsraum 8 strömt und so der zweite Arbeitskolben 2 nach vorne gedrückt werden beziehungsweise der erste Arbeitskolben 1 nach vorne gezogen werden (in den Figuren 1 bis 5 nach links). Hierdurch kann eine periodische lineare Bewegung beziehungsweise Oszillation der Arbeitskolben 1, 2 und des Ventilkolbens 6 erzeugt werden. Diese Bewegung wird durch das Vakuum beziehungsweise den Unterdruck als erster Druck und den umgebenden Luftdruck als zweiter Druck angetrieben. Alternativ kann auch der Vakuumanschluss 28 offen liegen und eine Druckgasquelle mit einem Druck größer als der umgebende Luftdruck an die vierte Öffnung 24 und die fünfte Öffnung 26 angeschlossen werden. Das Funktionsprinzip bleibt dabei das gleiche. Um eine ausreichende Leistung des Differenzdruckmotors zu erzielen, ist ein ausreichender Differenzdruck zwischen dem ersten Druck und dem zweiten Druck sicherzustellen. Zudem kann durch ein Vergrößern der Arbeitsflächen 3, 4 ebenfalls eine Vergrößerung der Leistung des Differenzdruckmotors erreicht werden.

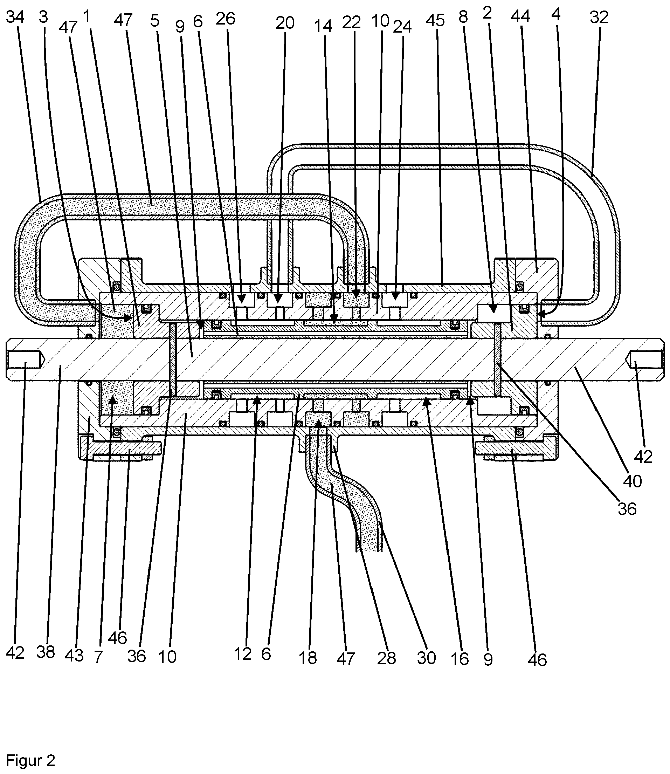

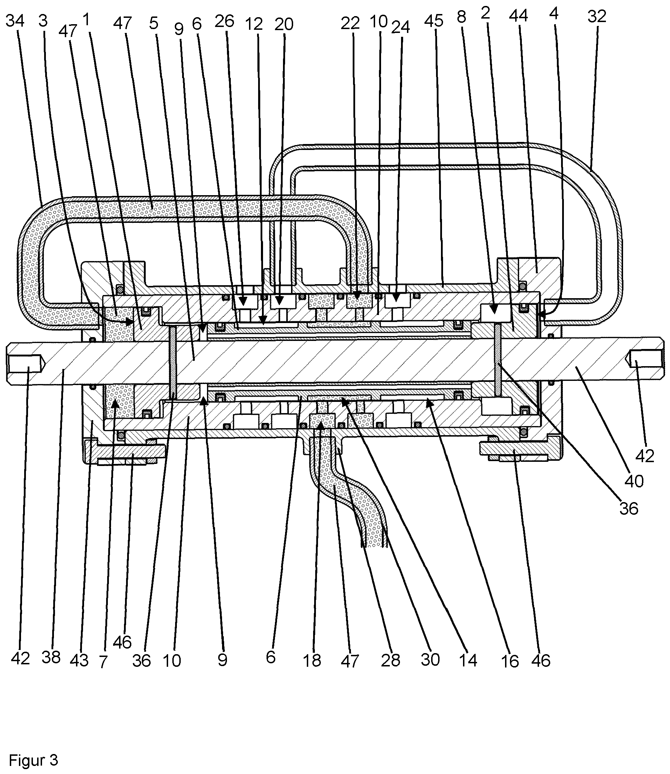

Im Ruhezustand des Differenzdruckmotors (siehe Figur 1) kann das Vakuum 47 an den Vakuumanschluss 28 angelegt werden (siehe Figur 2). Dabei kann der erste Arbeitsraum 3 evakuiert werden, während der zweite Arbeitsraum 4 nach außen offen ist. Aufgrund der Druckdifferenz zwischen dem ersten Arbeitsraum 3 und dem zweiten Arbeitsraum 4 können die Arbeitskolben 1, 2 mit der Stange 5 nach vorne getrieben werden, bis der zweite Arbeitskolben 2 im Ventilraum 9 auf den Ventilkolben 6 trifft (siehe Figur 3). Dabei kann der zweite Arbeitskolben 2 den Ventilkolben 6 nach vorne schlagen und so den Differenzdruckmotor in Gang setzen.

Die Arbeitskolben 1, 2 können über Bolzen 36 mit der Stange 5 verbunden sein. Hierdurch wird der Zusammenbau des Differenzdruckmotors vereinfacht. Die Stange 5 kann sich durch die beiden Arbeitskolben 1, 2 hindurch erstrecken. Hierdurch stehen an der Vorderseite des ersten Arbeitskolbens 1 eine erste Antriebsstange 38 und an der Rückseite des zweiten Arbeitskolbens 2 eine zweite Antriebsstange 40 vor, mit denen Werkzeuge oder eine Pumpe (in den Figuren 1 bis 5 nicht gezeigt) anzutreiben sind. Hierzu können an den Enden der Antriebsstangen 38, 40 je ein Befestigungselement 42 angeordnet sein. Das Befestigungselement 42 kann in Form eines Vierkantlochs oder einer Gewindebohrung vorliegen, das mit einem passenden Gegenbefestigungselement an einem Werkzeug verbindbar sein kann.

Der erste Arbeitsraum 3 kann an seiner Vorderseite mit einem Verschluss 43 aus Kunststoff verschlossen sein, der eine Stirnfläche des ersten Arbeitsraums 7 bildet. Die erste Antriebsstange 38 kann durch den Verschluss 43 hindurchgeführt sein. Der Verschluss 43 kann gegen die erste Antriebsstange 38 mit einem Dichtungsring abgedichtet sein, durch den die erste Antriebsstange 38 gleiten kann. Der zweite Arbeitsraum 8 kann an seiner Vorderseite mit einem Verschluss 44 aus Kunststoff verschlossen sein, der eine Stirnfläche des zweiten Arbeitsraums 8 bildet. Die zweite Antriebsstange 40 kann durch den Verschluss 44 hindurchgeführt sein. Der Verschluss 44 kann gegen die zweite Antriebsstange 40 mit einem Dichtungsring abgedichtet sein, durch den die zweite Antriebsstange 40 gleiten kann.

Der Differenzdruckmotor kann ferner eine Außenhülle 45 aus Kunststoff aufweisen, der die Wandungen 10 umschließt. Die Verschlüsse 43, 44 können mit Schrauben 46 auf die Außenhülle 45 geschraubt sein, wobei zur Abdichtung zwischen den Verschlüssen 43, 44 und der Außenhülle 45 jeweils ein Dichtungsring vorgesehen sein kann. Es kann vorgesehen sein, dass die Öffnungen 18, 20, 22, 24, 26 gegen die Außenhülle 45 mit Dichtungen abgedichtet sind. Die Außenhülle 45 kann auch den Vakuumanschluss 28 ausformen und vorzugsweise auch zum Ausformen von Anschlüssen zur Verbindung der zweiten Öffnung 20 mit der Leitung 32 zu dem zweiten Arbeitsraum 8 und zur Verbindung der dritten Öffnung 22 mit der Leitung 34 zu dem ersten Arbeitsraum 7 verwendet werden.

Die Figuren 6 und 7 zeigen ein erstes erfindungsgemäßes chirurgisches Antriebssystem und eine erfindungsgemäße medizinischen Vorrichtung mit einem zweiten erfindungsgemäßen Differenzdruckmotor, wobei der Differenzdruckmotor gemäß dem zweiten Ausführungsbeispiel im Unterschied zum Differenzdruckmotor gemäß dem ersten Ausführungsbeispiel nach den Figuren 1 bis 5 eine Starthilfe in Form eines gespannten Federelements 91 aufweisen kann, die ein problemloses Anlaufen des Differenzdruckmotors sicherstellt.

Der zweite beispielhafte erfindungsgemäße Differenzdruckmotor weist einen ersten Arbeitskolben 51 und einen zweiten Arbeitskolben 52 auf. Der erste Arbeitskolben 51 kann eine in Richtung der Vorderseite des Differenzdruckmotors (in den Figuren 6 und 7 links) weisende Arbeitsfläche 53 aufweisen. Analog dazu kann der zweite Arbeitskolben 52 eine in Richtung der Rückseite des Differenzdruckmotors (in den Figuren 6 und 7 rechts) weisende Arbeitsfläche 54 aufweisen. Die Arbeit an den beiden Arbeitskolben 51, 52 kann so auf jeweils gegenüberliegenden Seiten geleistet werden, so dass der Druckgasmotor beidseitig angetrieben werden kann. Der erste Arbeitskolben 51 und der zweite Arbeitskolben 52 können über eine Stange 55 fest miteinander verbunden sein. Die Stange 55 kann die beiden Arbeitskolben 51, 52 in einem festen Abstand zueinander halten. Ferner kann durch die Stange 55 die Ausrichtung der Arbeitskolben 51, 52 zueinander fixiert sein.

Zwischen den beiden Arbeitskolben 51, 52 kann um die Stange 55 herum ein hülsenförmiger Ventilkolben 56 angeordnet sein, der auf der Stange 55 axial (bezogen auf die Stange 55) beweglich gelagert ist. Der Ventilkolben 56 kann in seiner axialen Ausdehnung kleiner sein, als der durch die Stange 55 bestimmte Abstand der beiden Arbeitskolben 51, 52. Hierdurch kann der Ventilkolben 56 zwischen den beiden Arbeitskolben 51, 52 beweglich sein. Erfindungsgemäß bevorzugt kann vorgesehen sein, dass der Ventilkolben 56 bezogen auf die Länge der Stange 55 zwischen den beiden Arbeitskolben 51, 52, die den Abstand zwischen den beiden Arbeitskolben 51, 52 bestimmt, kleiner ist als der Abstand zwischen den beiden Arbeitskolben 51, 52. Hierdurch wird eine Impulsübertragung zwischen den von den Arbeitsflächen 53, 54 abgewandten Seiten der Arbeitskolben 51, 52 auf den Ventilkolben 56 mittels eines Stoßes möglich und die Massenträgheit des Ventilkolbens 56 kann dazu genutzt werden, den Ventilkolben 56 über einen Totpunkt eines mit dem Ventilkolbens 56 aufgebauten Ventils hinweg zu stoßen.

Die Arbeitskolben 51, 52, die Stange 55 und der Ventilkolben 56 können sich in einem bereichsweise zylindrischen Hohlraum befinden. Der erste Arbeitskolben 51 kann in einem ersten zylindrischen Arbeitsraum 57 angeordnet sein. Der zweite Arbeitskolben 52 kann in einem zweiten zylindrischen Arbeitsraum 58 angeordnet sein. Der Ventilkolben 56 kann in einem zylindrischen Ventilraum 59 angeordnet sein. Der erste zylindrische Arbeitsraum 57 und der zweite zylindrische Arbeitsraum 58 können einen größeren Durchmesser haben als der dazwischen angeordnete Ventilraum 59. Die Arbeitskolben 51, 52 können in den Arbeitsräumen 57, 58 in axialer Richtung beweglich angeordnet sein. Die Arbeitskolben 51, 52 können auf der der jeweiligen Arbeitsfläche 53, 54 gegenüberliegenden Seite einen Fortsatz mit geringerem Durchmesser aufweisen, der in den Ventilraum 59 hineinreicht. Dadurch können die Arbeitskolben 51, 52 mit ihren Fortsätzen im Ventilraum 59 auf den Ventilkolben 56 stoßen. Die Arbeitskolben 51, 52 können im Bereich der Arbeitsflächen 53, 54 einen zum Innenumfang des jeweiligen Arbeitsraums 57, 58 passenden Außenumfang aufweisen. Bevorzugt dichten die Arbeitskolben den jeweiligen Arbeitsraum 57, 58 gasdicht oder druckdicht ab. Hierzu können am Außenumfang der Arbeitskolben 51, 52 umlaufende Kolbenringe (siehe Figuren 6 und 7) oder andere Dichtungen vorgesehen sein.

Der Ventilkolben 56 kann außen eine zum zylindrischen Ventilraum 59 passende zylindrische Form aufweisen. Der Ventilkolben 56 kann am Außenumfang an seinen beiden den Arbeitskolben 51, 52 zugewandten Enden je einen umlaufenden Kolbenring (siehe Figuren 6 und 7) oder einen anderen Dichtungsring aufweisen. Im Inneren des Ventilkolbens 56 kann ein gasdurchlässiger Durchgang angeordnet sein, der die beiden den Arbeitskolben 51, 52 zugewandten Seiten des Ventilkolbens 56 gasdurchlässig miteinander verbindet.

Die beiden Arbeitsräume 57, 58 und der Ventilraum 59 bilden zusammen den bereichsweise zylindrischen Hohlraum, in dem die Arbeitskolben 51, 52 mit der Stange 55 und dem Ventilkolben 56 beweglich angeordnet sind. Der Hohlraum kann durch einen Hohlkörper aus Kunststoff geformt werden. Der Hohlraum kann durch Wandungen 60 des Hohlraums begrenzt sein.

In einem ansonsten zylindrischen Außenumfang des Ventilkolbens 56 können drei umlaufende rotationssymmetrische Nuten 62, 64, 66 angeordnet sein. Diese Nuten 62, 64, 66 können mit der sie umgebenden Wandung 60 drei voneinander getrennte ringförmige Hohlräume bilden. Diese können zum Schalten eines Ventils verwendet werden, das mit dem Ventilkolben 56 gebildet wird. Zur Bildung eines solchen Ventils können im Bereich des Ventilraums 59 fünf durchgehende Öffnungen 68, 70, 72, 74, 76 in der Wandung 60 angeordnet sein. Die fünf durchgehenden Öffnungen 68, 70, 72, 74, 76 können axial (bezogen auf den zylindrischen Ventilraum 59) nebeneinander angeordnet sein. Eine erste mittlere Öffnung 68 kann in einen Vakuumanschluss 78 münden. Eine Vakuumleitung 80 in Form eines Schlauchs kann an den Vakuumanschluss 78 angeschlossen sein. Die Vakuumleitung 80 kann den Vakuumanschluss 78 und damit die erste Öffnung 68 mit einer Vakuumquelle oder Unterdruckquelle verbinden. Eine zweite Öffnung 70, die axial neben der ersten Öffnung 68 angeordnet sein kann, kann über eine Leitung 82 gasdurchlässig mit dem zweiten Arbeitsraum 58 verbunden sein. Eine dritte Öffnung 72, die axial neben der ersten Öffnung 68 aber gegenüberliegend der zweiten Öffnung 70 angeordnet sein kann, kann über eine Leitung 84 gasdurchlässig mit dem ersten Arbeitsraum 57 verbunden sein. Eine vierte außen liegende Öffnung 74 kann gasdurchlässig mit der Umgebung des Differenzdruckmotors verbunden sein. Eine fünfte außen liegende Öffnung 76 kann gasdurchlässig mit der Umgebung des Differenzdruckmotors verbunden sein.

Die axial nebeneinanderliegenden durchgehenden Öffnungen 68, 70, 72, 74, 76 können mit der Wandung 60 und dem axial innerhalb des Ventilraums 59 beweglichen und gegen die Wandung 60 abgedichteten Ventilkolben 56 und den Nuten 62, 64, 66 das Ventil bilden, mit dem der Differenzdruckmotor gesteuert wird. Die Bewegung des Ventilkolbens 56 kann dabei durch die Arbeitskolben 51, 52 angeregt werden, die von beiden Seiten auf den Ventilkolben 56 stoßen und so zu einer Oszillation anregen können. Die Nuten 62, 64, 66 können bevorzugt so breit sein, dass jeweils zwei benachbarte Öffnungen 68, 70, 72, 74, 76 gleichzeitig in eine der Nuten 62, 64, 66 münden. Hierdurch können je nach Stellung des Ventilkolbens 56 immer zwei Öffnungen 68, 70, 72, 74, 76 miteinander gasdurchlässig verbunden sein. Die Nuten 62, 64, 66 sind bevorzugt aber in axialer Richtung nicht so breit, dass drei Öffnungen 68, 70, 72, 74, 76 in die gleiche Nut 62, 64, 66 münden können. Ferner kann vorzugsweise die Wand zwischen den Nuten 62, 64, 66 in axialer Richtung so breit sein, dass sie zumindest die zweite Öffnung 70 und die dritte Öffnung 72 geradeso abdecken kann, so dass ausgeschlossen ist, dass die zweite Öffnung 70 oder die dritte Öffnung 72 gleichzeitig in zwei der Nuten 62, 64, 66 münden kann und so einen "Kurzschluss" des Differenzdruckmotors bewirkt. Aus diesem Grund ist es auch wichtig, dass der Ventilkolben 56, insbesondere aufgrund seiner Trägheit, automatisch den Punkt überfährt, bei dem die zweite Öffnung 70 oder die dritte Öffnung 72 geschlossen sind und in keine der Nuten 62, 64, 66 münden. Hierdurch wird eine Totstellung des Differenzdruckmotors vermieden.

In einer ersten Stellung des Ventilkolbens 56 kann die erste mittlere Öffnung 68 und die benachbarte zweite Öffnung 70 miteinander über die mittlere Nut 64 gasleitend verbunden sein und die dritte Öffnung 72 und die dazu benachbarte randseitige vierte Öffnung 74 durch die rückseitige Nut 66 miteinander gasleitend verbunden sein und die fünf Öffnungen 68, 70, 72, 74, 76 ansonsten durch das Ventil voneinander gasdicht getrennt sein. In einer zweiten Stellung des Ventilkolbens 56 (siehe Figur 6 und 7) kann die erste mittlere Öffnung 68 und die dritte Öffnung 72 miteinander über die mittlere Nut 64 gasleitend verbunden sein und die zweite Öffnung 70 und die dazu benachbarte randseitige fünfte Öffnung 76 durch die vorderseitige Nut 62 miteinander gasleitend verbunden sein und die fünf Öffnungen 68, 70, 72, 74, 76 ansonsten durch das Ventil voneinander gasdicht getrennt sein. Der Ventilkolben 56 kann durch die Arbeitskolben 51, 52 von der ersten Stellung in die zweite Stellung überführt werden. Bei Einwirken eines Vakuums am Vakuumanschluss 78 kann in der ersten Stellung des Ventilkolbens 56 der zweite Arbeitsraum 58 evakuiert werden. Gleichzeitig kann der erste Arbeitsraum 57 über die dritte Öffnung 72, die vierte Öffnung 74 und die hintere Nut 66 mit der Umgebung gasdurchlässig verbunden sein, so dass Luft in den ersten Arbeitsraum 57 strömt beziehungsweise vorhanden ist. Durch diese Druckdifferenz kann der erste Arbeitskolben 51 nach hinten gedrückt werden, beziehungsweise der zweite Arbeitskolben 52 nach hinten gezogen werden (in den Figuren 6 und 7 nach rechts). Wenn der erste Arbeitskolben 51, der mit dem zweiten Arbeitskolben 52 über die Stange 55 verbunden ist, nach hinten gezogen wird und trifft dieser auf den Ventilkolben 56. Der Ventilkolben 56 kann dadurch aus der ersten Stellung herausgestoßen werden und in die zweite Stellung überführt werden. In dieser zweiten Stellung des Ventilkolbens 56 (siehe Figur 6 und 7) kann der erste Arbeitsraum 57 evakuiert werden, während er von der Umgebung getrennt ist. Gleichzeitig kann der zweite Arbeitsraum 58 über die zweite Öffnung 70, die fünfte Öffnung 76 und die vordere Nut 62 mit der Umgebung gasdurchlässig verbunden sein, so dass Luft in den zweiten Arbeitsraum 58 strömt und so der zweite Arbeitskolben 52 nach vorne gedrückt werden beziehungsweise der erste Arbeitskolben 51 nach vorne gezogen werden (in den Figuren 6 und 7 nach links). Hierdurch kann eine periodische lineare Bewegung beziehungsweise Oszillation der Arbeitskolben 51, 52 und des Ventilkolbens 56 erzeugt werden. Diese Bewegung wird durch das Vakuum beziehungsweise den Unterdruck als erster Druck und den umgebenden Luftdruck als zweiter Druck angetrieben. Alternativ kann auch der Vakuumanschluss 78 offen liegen und eine Druckgasquelle mit einem Druck größer als der umgebende Luftdruck an die vierte Öffnung 74 und die fünfte Öffnung 76 angeschlossen werden. Das Funktionsprinzip bleibt dabei das gleiche. Um eine ausreichende Leistung des Differenzdruckmotors zu erzielen, ist ein ausreichender Differenzdruck zwischen dem ersten Druck und dem zweiten Druck sicherzustellen. Zudem kann durch ein Vergrößern der Arbeitsflächen 53, 54 ebenfalls eine Vergrößerung der Leistung des Differenzdruckmotors erreicht werden.

Im Ruhezustand des Differenzdruckmotors kann das Federelement 91, die als Druckfeder zwischen einem die Rückseite des zweiten Arbeitsraums 58 begrenzenden Verschluss 94 aus Kunststoff und einer vorstehenden Ringscheibe auf der Stange 55 angeordnet sein kann, die Stange 55 und die Arbeitskolben 51, 52 nach hinten ziehen. Dadurch wird auch der Ventilkolben 56 automatisch in eine die zweite Stellung gebracht. Damit wird verhindert, dass der Differenzdruckmotor in einem Totpunkt zum Stehen kommt und nicht wieder anspringen kann.

Die Arbeitskolben 51, 52 können über Bolzen 86 mit der Stange 55 verbunden sein. Um den Bolzen 86 für den ersten Arbeitskolben 51 problemlos montieren zu können, ist eine Durchführung vorgesehen, die nach der Montage durch zwei Stopfen 61 verschlossen werden kann. Hierdurch wird der Zusammenbau des Differenzdruckmotors vereinfacht. Die Stange 55 kann sich durch die beiden Arbeitskolben 51, 52 hindurch erstrecken. Hierdurch steht an der Vorderseite des ersten Arbeitskolbens 51 eine erste Antriebsstange 88 vor. Es kann auch vorgesehen sein, dass an der Rückseite des zweiten Arbeitskolbens 52 eine zweite Antriebsstange 90 vorsteht (siehe Figur 6). Auf die zweite Antriebsstange 90 kann aber auch verzichtet werden (siehe Figur 7). Mit den Antriebsstangen 88, 90 können Werkzeuge, wie beispielsweise eine Säge 102 (siehe Figur 7), oder eine Pumpe angetrieben werden. Hierzu können an den Enden der Antriebsstangen 88, 90 je ein Befestigungselement 92 angeordnet sein. Das Befestigungselement 92 kann in Form eines Vierkantlochs oder einer Gewindebohrung vorliegen, das mit einem passenden Gegenbefestigungselement an einem Werkzeug verbindbar sein kann.

Der erste Arbeitsraum 53 kann an seiner Vorderseite mit einem Verschluss 93 aus Kunststoff verschlossen sein, der eine Stirnfläche des ersten Arbeitsraums 57 bildet. Die erste Antriebsstange 88 kann durch den Verschluss 93 hindurchgeführt sein. Der Verschluss 93 kann gegen die erste Antriebsstange 88 mit einem Dichtungsring abgedichtet sein, durch den die erste Antriebsstange 88 gleiten kann. Der zweite Arbeitsraum 58 kann an seiner Vorderseite mit dem Verschluss 94 verschlossen sein, der eine Stirnfläche des zweiten Arbeitsraums 58 bildet. Die zweite Antriebsstange 90 kann durch den Verschluss 94 hindurchgeführt sein. Der Verschluss 94 kann gegen die zweite Antriebsstange 90 mit einem Dichtungsring abgedichtet sein, durch den die zweite Antriebsstange 90 gleiten kann.

Der Differenzdruckmotor kann ferner eine Außenhülle 95 aus Kunststoff aufweisen, der die Wandungen 60 umschließt. Die Verschlüsse 93, 94 können mit Schrauben 96 auf die Außenhülle 95 geschraubt sein, wobei zur Abdichtung zwischen den Verschlüssen 93, 94 und der Außenhülle 95jeweils ein Dichtungsring vorgesehen sein kann. Es kann vorgesehen sein, dass die Öffnungen 68, 70, 72, 74, 76 gegen die Außenhülle 95 mit Dichtungen abgedichtet sind. Die Außenhülle 95 kann auch den Vakuumanschluss 78 ausformen und vorzugsweise auch zum Ausformen von Anschlüssen zur Verbindung der zweiten Öffnung 70 mit der Leitung 82 zu dem zweiten Arbeitsraum 58 und zur Verbindung der dritten Öffnung 72 mit der Leitung 84 zu dem ersten Arbeitsraum 57 verwendet werden.

In der Vakuumleitung 80 des chirurgischen Antriebssystems nach Figur 6 beziehungsweise der medizinischen Vorrichtung nach Figur 7 kann ein manuell bedienbares Ventilelement 81 angeordnet sein, mit dem die Verbindung zwischen dem Vakuumanschluss 78 und der Vakuumquelle beziehungsweise der Unterdruckquelle unterbrochen werden kann. Das Ventilelement 81 kann einen Ventilkörper 97 aufweisen, der in einem Ventilgehäuse 98 linear verschiebbar gelagert ist. Eine Feder 99 im Ventilgehäuse 98 kann den Ventilkörper 97 in eine geschlossene Stellung drücken. Mit einem Abzug 100 kann der Ventilkörper 97 gegen die Feder 99 in die geöffnete Stellung drücken und so der Differenzdruckmotor zum Laufen gebracht werden.

Die erfindungsgemäße medizinische Vorrichtung zum Bürsten, Raspeln oder Sägen von Weichgewebe und/oder Knochengewebe nach Figur 7 kann zusätzlich noch ein Gehäuse 104 aus Kunststoff aufweisen, in dem der Differenzdruckmotor und das Ventilelement 81 angeordnet sind. Das Gehäuse 104 kann an seiner Unterseite (in Figur 7 unten) als Griff 106 ausgeformt sein. Die Vorrichtung kann dann am Griff 106 in einer Hand gehalten und mit der gleichen Hand der Abzug 100 bedient werden.

Die Figur 8 zeigt eine schematische Querschnittansicht eines dritten beispielhaften erfindungsgemäßen Differenzdruckmotors während des Betriebs mit angelegtem Druckgas 197. Mit dem mit Druckgas 197 angetriebenen Differenzdruckmotor kann ein chirurgisches Antriebssystem aufgebaut werden, mit dem wiederum eine medizinische Vorrichtung analog dem Ausführungsbeispiel nach Figur 7 aufgebaut wird. Hierzu muss lediglich ein Ventil (analog dem Ventilelement 81 nach den Figuren 6 und 7, in Figur 8 aber nicht gezeigt) in eine Druckgasleitung 180 eingebaut werden, über die das Druckgas 197 als Antriebsfluid in den Differenzdruckmotor eingespeist wird. Der Differenzdruckmotor nach Figur 8 weist keine Starthilfe auf, könnte aber ohne weiteres mit einer solchen ausgeführt werden.

Der dritte beispielhafte erfindungsgemäße Differenzdruckmotor weist einen ersten Arbeitskolben 151 und einen zweiten Arbeitskolben 152 auf. Der erste Arbeitskolben 151 kann eine in Richtung der Vorderseite des Differenzdruckmotors (in Figur 8 links) weisende Arbeitsfläche 153 aufweisen. Analog dazu kann der zweite Arbeitskolben 152 eine in Richtung der Rückseite des Differenzdruckmotors (in Figur 8 rechts) weisende Arbeitsfläche 154 aufweisen. Die Arbeit an den beiden Arbeitskolben 151, 152 kann so auf jeweils gegenüberliegenden Seiten geleistet werden, so dass der Druckgasmotor beidseitig angetrieben werden kann. Der erste Arbeitskolben 151 und der zweite Arbeitskolben 152 können über eine Stange 155 fest miteinander verbunden sein. Die Stange 155 kann die beiden Arbeitskolben 151, 152 in einem festen Abstand zueinander halten. Ferner kann durch die Stange 155 die Ausrichtung der Arbeitskolben 151, 152 zueinander fixiert sein.

Zwischen den beiden Arbeitskolben 151, 152 kann um die Stange 155 herum ein hülsenförmiger Ventilkolben 156 angeordnet sein, der auf der Stange 155 axial (bezogen auf die Stange 155) beweglich gelagert ist. Der Ventilkolben 156 kann in seiner axialen Ausdehnung kleiner sein, als der durch die Stange 155 bestimmte Abstand der beiden Arbeitskolben 151, 152. Hierdurch kann der Ventilkolben 156 zwischen den beiden Arbeitskolben 151, 152 beweglich sein. Erfindungsgemäß bevorzugt kann vorgesehen sein, dass der Ventilkolben 156 bezogen auf die Länge der Stange 155 zwischen den beiden Arbeitskolben 151, 152, die den Abstand zwischen den beiden Arbeitskolben 151, 152 bestimmt, kleiner ist als der Abstand zwischen den beiden Arbeitskolben 151, 152. Hierdurch wird eine Impulsübertragung zwischen den von den Arbeitsflächen 153, 154 abgewandten Seiten der Arbeitskolben 151, 152 auf den Ventilkolben 156 mittels eines Stoßes möglich und die Massenträgheit des Ventilkolbens 156 kann dazu genutzt werden, den Ventilkolben 156 über einen Totpunkt eines mit dem Ventilkolbens 156 aufgebauten Ventils hinweg zu stoßen.

Die Arbeitskolben 151, 152, die Stange 155 und der Ventilkolben 156 können sich in einem bereichsweise zylindrischen Hohlraum befinden. Der erste Arbeitskolben 151 kann in einem ersten zylindrischen Arbeitsraum 157 angeordnet sein. Der zweite Arbeitskolben 152 kann in einem zweiten zylindrischen Arbeitsraum 158 angeordnet sein. Der Ventilkolben 156 kann in einem zylindrischen Ventilraum 159 angeordnet sein. Der erste zylindrische Arbeitsraum 157 und der zweite zylindrische Arbeitsraum 158 können einen größeren Durchmesser haben als der dazwischen angeordnete Ventilraum 159. Die Arbeitskolben 151, 152 können in den Arbeitsräumen 157, 158 in axialer Richtung beweglich angeordnet sein. Die Arbeitskolben 151, 152 können auf der der jeweiligen Arbeitsfläche 153, 154 gegenüberliegenden Seite einen Fortsatz mit geringerem Durchmesser aufweisen, der in den Ventilraum 159 hineinreicht. Dadurch können die Arbeitskolben 151, 152 mit ihren Fortsätzen im Ventilraum 159 auf den Ventilkolben 156 stoßen. Die Arbeitskolben 151, 152 können im Bereich der Arbeitsflächen 153, 154 einen zum Innenumfang des jeweiligen Arbeitsraums 157, 158 passenden Außenumfang aufweisen. Bevorzugt dichten die Arbeitskolben den jeweiligen Arbeitsraum 157, 158 gasdicht oder druckdicht ab. Hierzu können am Außenumfang der Arbeitskolben 151, 152 umlaufende Kolbenringe (siehe Figur 8) oder andere Dichtungen vorgesehen sein.

Der Ventilkolben 156 kann außen eine zum zylindrischen Ventilraum 159 passende zylindrische Form aufweisen. Der Ventilkolben 156 kann am Außenumfang an seinen beiden den Arbeitskolben 151, 152 zugewandten Enden je einen umlaufenden Kolbenring (siehe Figur 8) oder einen anderen Dichtungsring aufweisen. Im Inneren des Ventilkolbens 156 kann ein gasdurchlässiger Durchgang angeordnet sein, der die beiden den Arbeitskolben 151, 152 zugewandten Seiten des Ventilkolbens 156 gasdurchlässig miteinander verbindet.

Die beiden Arbeitsräume 157, 158 und der Ventilraum 159 bilden zusammen den bereichsweise zylindrischen Hohlraum, in dem die Arbeitskolben 151, 152 mit der Stange 155 und dem Ventilkolben 156 beweglich angeordnet sind. Der Hohlraum kann durch einen Hohlkörper aus Kunststoff geformt werden. Der Hohlraum kann durch Wandungen 160 des Hohlraums begrenzt sein.

In einem ansonsten zylindrischen Außenumfang des Ventilkolbens 156 können drei umlaufende rotationssymmetrische Nuten 162, 164, 166 angeordnet sein. Diese Nuten 162, 164, 166 können mit der sie umgebenden Wandung 160 drei voneinander getrennte ringförmige Hohlräume bilden. Diese können zum Schalten eines Ventils verwendet werden, das mit dem Ventilkolben 156 gebildet wird. Zur Bildung eines solchen Ventils können im Bereich des Ventilraums 159 fünf durchgehende Öffnungen 168, 170, 172, 174, 176 in der Wandung 160 angeordnet sein. Die fünf durchgehenden Öffnungen 168, 170, 172, 174, 176 können axial (bezogen auf den zylindrischen Ventilraum 159) nebeneinander angeordnet sein. Eine erste mittlere Öffnung 168 kann in einen Druckgasanschluss 178 münden. Die Druckgasleitung 180 in Form eines Schlauchs kann an den Druckgasanschluss 178 angeschlossen sein. Die Druckgasleitung 180 kann den Druckgasanschluss 178 und damit die erste Öffnung 168 mit einer Druckgasquelle verbinden. Eine zweite Öffnung 170, die axial neben der ersten Öffnung 168 angeordnet sein kann, kann über eine Leitung 184 gasdurchlässig mit dem ersten Arbeitsraum 157 verbunden sein. Eine dritte Öffnung 172, die axial neben der ersten Öffnung 168 aber gegenüberliegend der zweiten Öffnung 170 angeordnet sein kann, kann über eine Leitung 182 gasdurchlässig mit dem zweiten Arbeitsraum 158 verbunden sein. Damit sind bei dem mit Druckgas angetriebenen dritten erfindungsgemäßen Differenzdruckmotor die Verbindungen der zweiten Öffnung 170 und der dritten Öffnung 172 mit den Arbeitsräumen 157, 158 getauscht im Vergleich zum ersten und zweiten Ausführungsbeispiel. Eine vierte außen liegende Öffnung 174 kann gasdurchlässig mit der Umgebung des Differenzdruckmotors verbunden sein. Eine fünfte außen liegende Öffnung 176 kann gasdurchlässig mit der Umgebung des Differenzdruckmotors verbunden sein. Anstelle den Differenzdruck zwischen einem Vakuum oder Unterdruck und der Umgebungsluft zu nutzen, wird also bei dem vorliegenden dritten Ausführungsbeispiel der Differenzdruck zwischen einem Druckgas 197 und der Umgebungsluft genutzt.

Die axial nebeneinanderliegenden durchgehenden Öffnungen 168, 170, 172, 174, 176 können mit der Wandung 160 und dem axial innerhalb des Ventilraums 159 beweglichen und gegen die Wandung 160 abgedichteten Ventilkolben 156 und den Nuten 162, 164, 166 das Ventil bilden, mit dem der Differenzdruckmotor gesteuert wird. Die Bewegung des Ventilkolbens 156 kann dabei durch die Arbeitskolben 151, 152 angeregt werden, die von beiden Seiten auf den Ventilkolben 156 stoßen und so zu einer Oszillation anregen können. Die Nuten 162, 164, 166 können bevorzugt so breit sein, dass jeweils zwei benachbarte Öffnungen 168, 170, 172, 174, 176 gleichzeitig in eine der Nuten 162, 164, 166 münden. Hierdurch können je nach Stellung des Ventilkolbens 156 immer zwei Öffnungen 168, 170, 172, 174, 176 miteinander gasdurchlässig verbunden sein. Die Nuten 162, 164, 166 sind bevorzugt aber in axialer Richtung nicht so breit, dass drei Öffnungen 168, 170, 172, 174, 176 in die gleiche Nut 162, 164, 166 münden können. Ferner kann vorzugsweise die Wand zwischen den Nuten 162, 164, 166 in axialer Richtung so breit sein, dass sie zumindest die zweite Öffnung 170 und die dritte Öffnung 172 geradeso abdecken kann, so dass ausgeschlossen ist, dass die zweite Öffnung 170 oder die dritte Öffnung 172 gleichzeitig in zwei der Nuten 162, 164, 166 münden kann und so einen "Kurzschluss" des Differenzdruckmotors bewirkt. Aus diesem Grund ist es auch wichtig, dass der Ventilkolben 156, insbesondere aufgrund seiner Trägheit, automatisch den Punkt überfährt, bei dem die zweite Öffnung 170 oder die dritte Öffnung 172 geschlossen sind und in keine der Nuten 162, 164, 166 münden. Hierdurch wird eine Totstellung des Differenzdruckmotors vermieden.

In einer ersten Stellung des Ventilkolbens 156 kann die erste mittlere Öffnung 168 und die benachbarte zweite Öffnung 170 miteinander über die mittlere Nut 164 gasleitend verbunden sein und die dritte Öffnung 172 und die dazu benachbarte randseitige vierte Öffnung 174 durch die rückseitige Nut 166 miteinander gasleitend verbunden sein und die fünf Öffnungen 168, 170, 172, 174, 176 ansonsten durch das Ventil voneinander gasdicht getrennt sein (nicht die Figur 8 dargestellte Position). In einer zweiten Stellung des Ventilkolbens 156, die in Figur 8 dargestellt ist, kann die erste mittlere Öffnung 168 und die dritte Öffnung 172 miteinander über die mittlere Nut 164 gasleitend verbunden sein und die zweite Öffnung 170 und die dazu benachbarte randseitige fünfte Öffnung 176 durch die vorderseitige Nut 162 miteinander gasleitend verbunden sein und die fünf Öffnungen 168, 170, 172, 174, 176 ansonsten durch das Ventil voneinander gasdicht getrennt sein. Der Ventilkolben 156 kann durch die Arbeitskolben 151, 152 von der ersten Stellung in die zweite Stellung überführt werden. Bei Einwirken des Druckgases 197 am Druckgasanschluss 178 kann in der ersten Stellung des Ventilkolbens 156 der erste Arbeitsraum 157 mit dem Druckgas 197 gefüllt werden. Gleichzeitig kann der zweite Arbeitsraum 158 über die dritte Öffnung 172, die vierte Öffnung 174 und die hintere Nut 166 mit der Umgebung gasdurchlässig verbunden sein, so dass in dem zweiten Arbeitsraum 158 enthaltenes Druckgas 197 aus dem zweiten Arbeitsraum 158 herausströmt und an die Umgebung abbläst. Durch diese Druckdifferenz kann der erste Arbeitskolben 151 nach hinten gedrückt werden, beziehungsweise der zweite Arbeitskolben 152 nach hinten gezogen werden (in Figur 8 nach rechts). Wenn der erste Arbeitskolben 151, der mit dem zweiten Arbeitskolben 152 über die Stange 155 verbunden ist, nach hinten gezogen wird und trifft dieser auf den Ventilkolben 156. Der Ventilkolben 156 kann dadurch aus der ersten Stellung herausgestoßen werden und in die zweite Stellung überführt werden. In dieser zweiten Stellung des Ventilkolbens 156 (siehe Figur 8) kann das Druckgas 197 in den zweiten Arbeitsraum 158 eingespeist werden, während er von der Umgebung getrennt ist. Gleichzeitig kann der erste Arbeitsraum 157 über die zweite Öffnung 170, die fünfte Öffnung 176 und die vordere Nut 162 mit der Umgebung gasdurchlässig verbunden sein, so dass das in dem ersten Arbeitsraum 157 vorhandene Druckgas 197 aus dem ersten Arbeitsraum 157 herausströmt und so der zweite Arbeitskolben 152 nach vorne gedrückt werden beziehungsweise der erste Arbeitskolben 151 nach vorne gezogen werden (in Figur 8 nach links). Hierdurch kann eine periodische lineare Bewegung beziehungsweise Oszillation der Arbeitskolben 151, 152 und des Ventilkolbens 156 erzeugt werden. Diese Bewegung wird durch den durch das Druckgas 197 bereitgestellten ersten Druck und den umgebenden Luftdruck als zweiten Druck angetrieben. Alternativ kann auch der Druckgasanschluss 178 offen liegen und eine Vakuumquelle oder eine Unterdruckquelle mit einem Druck kleiner als der umgebende Luftdruck an die vierte Öffnung 174 und die fünfte Öffnung 176 angeschlossen werden. Das Funktionsprinzip bleibt dabei das gleiche. Um eine ausreichende Leistung des Differenzdruckmotors zu erzielen, ist ein ausreichender Differenzdruck zwischen dem ersten Druck und dem zweiten Druck sicherzustellen. Zudem kann durch ein Vergrößern der Arbeitsflächen 153, 154 ebenfalls eine Vergrößerung der Leistung des Differenzdruckmotors erreicht werden.

Im Ruhezustand des Differenzdruckmotors eine Feder (in Figur 8 nicht gezeigt), die als Druckfeder zwischen einem die Rückseite des zweiten Arbeitsraums 158 begrenzenden Verschluss 194 aus Kunststoff und einer vorstehenden Ringscheibe (nicht gezeigt) auf der Stange 155 angeordnet sein kann, die Stange 155 und die Arbeitskolben 151, 152 nach hinten ziehen. Dadurch wird auch der Ventilkolben 156 automatisch in eine die zweite Stellung gebracht. Damit wird verhindert, dass der Differenzdruckmotor in einem Totpunkt zum Stehen kommt und nicht wieder anspringen kann.

Die Arbeitskolben 151, 152 können über Bolzen 186 mit der Stange 155 verbunden sein. Hierdurch wird der Zusammenbau des Differenzdruckmotors vereinfacht. Die Stange 155 kann sich durch die beiden Arbeitskolben 151, 152 hindurch erstrecken. Hierdurch steht an der Vorderseite des ersten Arbeitskolbens 151 eine erste Antriebsstange 188 vor. Es kann auch vorgesehen sein, dass an der Rückseite des zweiten Arbeitskolbens 152 eine zweite Antriebsstange 190 vorsteht. Auf die zweite Antriebsstange 190 kann aber auch theoretisch verzichtet werden, wenn nur ein einziger Antrieb benötigt wird. Mit den Antriebsstangen 188, 190 können Werkzeuge, wie beispielsweise eine Säge, eine Raspel, eine Bürste oder eine Pumpe angetrieben werden. Hierzu können an den Enden der Antriebsstangen 188, 190 je ein Befestigungselement 192 angeordnet sein. Das Befestigungselement 192 kann in Form eines Vierkantlochs oder einer Gewindebohrung vorliegen, das mit einem passenden Gegenbefestigungselement an einem Werkzeug verbindbar sein kann.

Der erste Arbeitsraum 153 kann an seiner Vorderseite mit einem Verschluss 193 aus Kunststoff verschlossen sein, der eine Stirnfläche des ersten Arbeitsraums 157 bildet. Die erste Antriebsstange 188 kann durch den Verschluss 193 hindurchgeführt sein. Der Verschluss 193 kann gegen die erste Antriebsstange 188 mit einem Dichtungsring abgedichtet sein, durch den die erste Antriebsstange 188 gleiten kann. Der zweite Arbeitsraum 158 kann an seiner Vorderseite mit dem Verschluss 194 verschlossen sein, der eine Stirnfläche des zweiten Arbeitsraums 158 bildet. Die zweite Antriebsstange 190 kann durch den Verschluss 194 hindurchgeführt sein. Der Verschluss 194 kann gegen die zweite Antriebsstange 190 mit einem Dichtungsring abgedichtet sein, durch den die zweite Antriebsstange 190 gleiten kann.

Der Differenzdruckmotor kann ferner eine Außenhülle 195 aus Kunststoff aufweisen, der die Wandungen 160 umschließt. Die Verschlüsse 193, 194 können mit Schrauben 196 auf die Außenhülle 195 geschraubt sein, wobei zur Abdichtung zwischen den Verschlüssen 193, 194 und der Außenhülle 195 jeweils ein Dichtungsring vorgesehen sein kann. Es kann vorgesehen sein, dass die Öffnungen 168, 170, 172, 174, 176 gegen die Außenhülle 195 mit Dichtungen abgedichtet sind. Die Außenhülle 195 kann auch den Druckgasanschluss 178 ausformen und vorzugsweise auch zum Ausformen von Anschlüssen zur Verbindung der zweiten Öffnung 170 mit der Leitung 184 zu dem ersten Arbeitsraum 157 und zur Verbindung der dritten Öffnung 172 mit der Leitung 182 zu dem zweiten Arbeitsraum 158 verwendet werden.

In der Druckgasleitung 180 des Differenzdruckmotors nach Figur 8 kann ein manuell bedienbares Ventilelement (nicht gezeigt) angeordnet sein, mit dem die Verbindung zwischen dem Druckgasanschluss 178 und der Druckgasquelle unterbrochen werden kann.

Die Figuren 9 bis 11 zeigen ein zweites erfindungsgemäßes chirurgisches Antriebssystem mit einem vierten beispielhaften erfindungsgemäßen Differenzdruckmotor, wobei der Differenzdruckmotor gemäß dem vierten Ausführungsbeispiel im Unterschied zum Differenzdruckmotor gemäß dem ersten Ausführungsbeispiel nach den Figuren 1 bis 5 eine Starthilfe in Form einer geeigneten Verbindung zur Vakuumquelle beziehungsweise Unterdruckquelle aufweisen kann, die ein problemloses Anlaufen des Differenzdruckmotors sicherstellt.

Der vierte beispielhafte erfindungsgemäße Differenzdruckmotor weist einen ersten Arbeitskolben 251 und einen zweiten Arbeitskolben 252 auf. Der erste Arbeitskolben 251 kann eine in Richtung der Vorderseite des Differenzdruckmotors (in den Figuren 9 bis 11 links) weisende Arbeitsfläche 253 aufweisen. Analog dazu kann der zweite Arbeitskolben 252 eine in Richtung der Rückseite des Differenzdruckmotors (in den Figuren 9 bis 11 rechts) weisende Arbeitsfläche 254 aufweisen. Die Arbeit an den beiden Arbeitskolben 251, 252 kann so auf jeweils gegenüberliegenden Seiten geleistet werden, so dass der Druckgasmotor beidseitig angetrieben werden kann. Der erste Arbeitskolben 251 und der zweite Arbeitskolben 252 können über eine Stange 255 fest miteinander verbunden sein. Die Stange 255 kann die beiden Arbeitskolben 251, 252 in einem festen Abstand zueinander halten. Ferner kann durch die Stange 255 die Ausrichtung der Arbeitskolben 251, 252 zueinander fixiert sein.

Zwischen den beiden Arbeitskolben 251, 252 kann um die Stange 255 herum ein hülsenförmiger Ventilkolben 256 angeordnet sein, der auf der Stange 255 axial (bezogen auf die Stange 255) beweglich gelagert ist. Der Ventilkolben 256 kann in seiner axialen Ausdehnung kleiner sein, als der durch die Stange 255 bestimmte Abstand der beiden Arbeitskolben 251, 252. Hierdurch kann der Ventilkolben 256 zwischen den beiden Arbeitskolben 251, 252 beweglich sein. Erfindungsgemäß bevorzugt kann vorgesehen sein, dass der Ventilkolben 256 bezogen auf die Länge der Stange 255 zwischen den beiden Arbeitskolben 251, 252, die den Abstand zwischen den beiden Arbeitskolben 251, 252 bestimmt, kleiner ist als der Abstand zwischen den beiden Arbeitskolben 251, 252. Hierdurch wird eine Impulsübertragung zwischen den von den Arbeitsflächen 253, 254 abgewandten Seiten der Arbeitskolben 251, 252 auf den Ventilkolben 256 mittels eines Stoßes möglich und die Massenträgheit des Ventilkolbens 256 kann dazu genutzt werden, den Ventilkolben 256 über einen Totpunkt eines mit dem Ventilkolbens 256 aufgebauten Ventils hinweg zu stoßen.

Die Arbeitskolben 251, 252, die Stange 255 und der Ventilkolben 256 können sich in einem bereichsweise zylindrischen Hohlraum befinden. Der erste Arbeitskolben 251 kann in einem ersten zylindrischen Arbeitsraum 257 angeordnet sein. Der zweite Arbeitskolben 252 kann in einem zweiten zylindrischen Arbeitsraum 258 angeordnet sein. Der Ventilkolben 256 kann in einem zylindrischen Ventilraum 259 angeordnet sein. Der erste zylindrische Arbeitsraum 257 und der zweite zylindrische Arbeitsraum 258 können einen größeren Durchmesser haben als der dazwischen angeordnete Ventilraum 259. Die Arbeitskolben 251, 252 können in den Arbeitsräumen 257, 258 in axialer Richtung beweglich angeordnet sein. Die Arbeitskolben 251, 252 können auf der der jeweiligen Arbeitsfläche 253, 254 gegenüberliegenden Seite einen Fortsatz mit geringerem Durchmesser aufweisen, der in den Ventilraum 259 hineinreicht. Dadurch können die Arbeitskolben 251, 252 mit ihren Fortsätzen im Ventilraum 259 auf den Ventilkolben 256 stoßen. Die Arbeitskolben 251, 252 können im Bereich der Arbeitsflächen 253, 254 einen zum Innenumfang des jeweiligen Arbeitsraums 257, 258 passenden Außenumfang aufweisen. Bevorzugt dichten die Arbeitskolben den jeweiligen Arbeitsraum 257, 258 gasdicht oder druckdicht ab. Hierzu können am Außenumfang der Arbeitskolben 251, 252 umlaufende Kolbenringe (siehe Figuren 9 bis 11) oder andere Dichtungen vorgesehen sein.

Der Ventilkolben 256 kann außen eine zum zylindrischen Ventilraum 259 passende zylindrische Form aufweisen. Der Ventilkolben 256 kann am Außenumfang an seinen beiden den Arbeitskolben 251, 252 zugewandten Enden je einen umlaufenden Kolbenring (siehe Figuren 9 bis 11) oder einen anderen Dichtungsring aufweisen. Im Inneren des Ventilkolbens 256 kann ein gasdurchlässiger Durchgang angeordnet sein, der die beiden den Arbeitskolben 251, 252 zugewandten Seiten des Ventilkolbens 256 gasdurchlässig miteinander verbindet.

Die beiden Arbeitsräume 257, 258 und der Ventilraum 259 bilden zusammen den bereichsweise zylindrischen Hohlraum, in dem die Arbeitskolben 251, 252 mit der Stange 255 und dem Ventilkolben 256 beweglich angeordnet sind. Der Hohlraum kann durch einen Hohlkörper aus Kunststoff geformt werden. Der Hohlraum kann durch Wandungen 260 des Hohlraums begrenzt sein.

In einem ansonsten zylindrischen Außenumfang des Ventilkolbens 256 können drei umlaufende rotationssymmetrische Nuten 262, 264, 266 angeordnet sein. Diese Nuten 262, 264, 266 können mit der sie umgebenden Wandung 260 drei voneinander getrennte ringförmige Hohlräume bilden. Diese können zum Schalten eines Ventils verwendet werden, das mit dem Ventilkolben 256 gebildet wird. Zur Bildung eines solchen Ventils können im Bereich des Ventilraums 259 fünf durchgehende Öffnungen 268, 270, 272, 274, 276 in der Wandung 260 angeordnet sein. Die fünf durchgehenden Öffnungen 268, 270, 272, 274, 276 können axial (bezogen auf den zylindrischen Ventilraum 259) nebeneinander angeordnet sein. Eine erste mittlere Öffnung 268 kann in einen Vakuumanschluss 278 münden. Eine Vakuumleitung 280 in Form eines Schlauchs kann an den Vakuumanschluss 278 angeschlossen sein. Die Vakuumleitung 280 kann den Vakuumanschluss 278 und damit die erste Öffnung 268 mit einer Vakuumquelle oder Unterdruckquelle verbinden. Eine zweite Öffnung 270, die axial neben der ersten Öffnung 268 angeordnet sein kann, kann über eine Leitung 282 gasdurchlässig mit dem zweiten Arbeitsraum 258 verbunden sein. Eine dritte Öffnung 272, die axial neben der ersten Öffnung 268 aber gegenüberliegend der zweiten Öffnung 270 angeordnet sein kann, kann über eine Leitung 284 gasdurchlässig mit dem ersten Arbeitsraum 257 verbunden sein. Eine vierte außen liegende Öffnung 274 kann gasdurchlässig mit der Umgebung des Differenzdruckmotors verbunden sein. Eine fünfte außen liegende Öffnung 276 kann gasdurchlässig mit der Umgebung des Differenzdruckmotors verbunden sein.

Die axial nebeneinanderliegenden durchgehenden Öffnungen 268, 270, 272, 274, 276 können mit der Wandung 260 und dem axial innerhalb des Ventilraums 259 beweglichen und gegen die Wandung 260 abgedichteten Ventilkolben 256 und den Nuten 262, 264, 266 das Ventil bilden, mit dem der Differenzdruckmotor gesteuert wird. Die Bewegung des Ventilkolbens 256 kann dabei durch die Arbeitskolben 251, 252 angeregt werden, die von beiden Seiten auf den Ventilkolben 256 stoßen und so zu einer Oszillation anregen können. Die Nuten 262, 264, 266 können bevorzugt so breit sein, dass jeweils zwei benachbarte Öffnungen 268, 270, 272, 274, 276 gleichzeitig in eine der Nuten 262, 264, 266 münden. Hierdurch können je nach Stellung des Ventilkolbens 256 immer zwei Öffnungen 268, 270, 272, 274, 276 miteinander gasdurchlässig verbunden sein. Die Nuten 262, 264, 266 sind bevorzugt aber in axialer Richtung nicht so breit, dass drei Öffnungen 268, 270, 272, 274, 276 in die gleiche Nut 262, 264, 266 münden können. Ferner kann vorzugsweise die Wand zwischen den Nuten 262, 264, 266 in axialer Richtung so breit sein, dass sie zumindest die zweite Öffnung 270 und die dritte Öffnung 272 geradeso abdecken kann, so dass ausgeschlossen ist, dass die zweite Öffnung 270 oder die dritte Öffnung 272 gleichzeitig in zwei der Nuten 262, 264, 266 münden kann und so einen "Kurzschluss" des Differenzdruckmotors bewirkt. Aus diesem Grund ist es auch wichtig, dass der Ventilkolben 256, insbesondere aufgrund seiner Trägheit, automatisch den Punkt überfährt, bei dem die zweite Öffnung 270 oder die dritte Öffnung 272 geschlossen sind und in keine der Nuten 262, 264, 266 münden. Hierdurch wird eine Totstellung des Differenzdruckmotors vermieden.

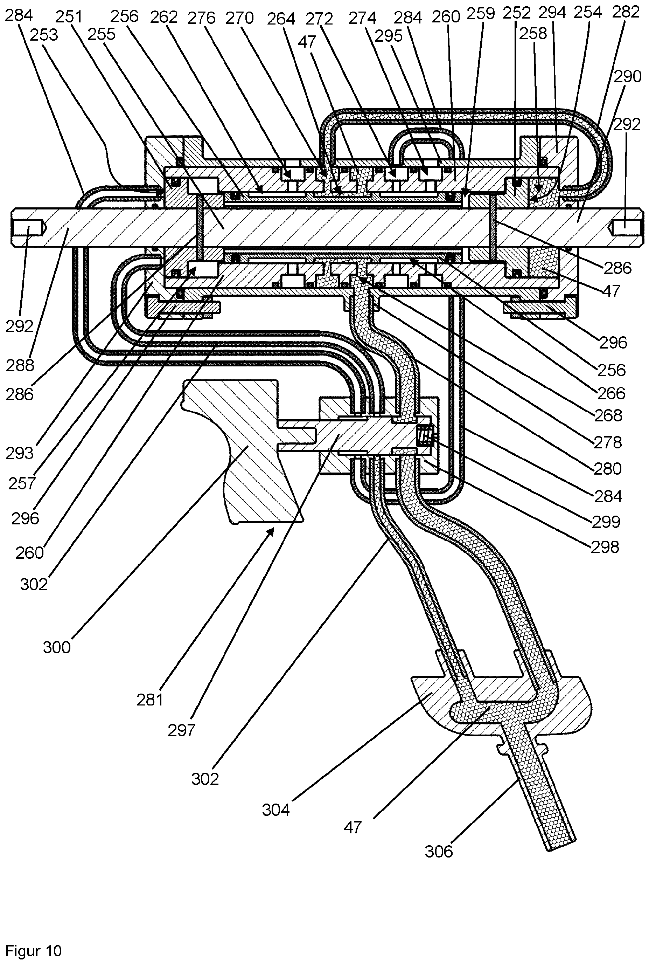

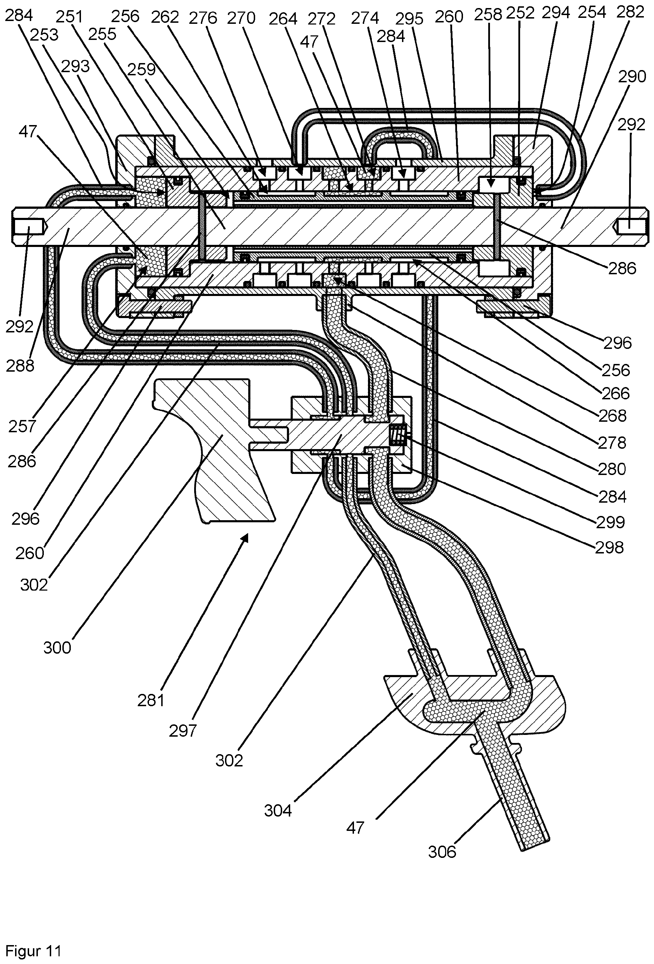

In einer ersten Stellung des Ventilkolbens 256 kann die erste mittlere Öffnung 268 und die benachbarte zweite Öffnung 270 miteinander über die mittlere Nut 264 gasleitend verbunden sein und die dritte Öffnung 272 und die dazu benachbarte randseitige vierte Öffnung 274 durch die rückseitige Nut 66 miteinander gasleitend verbunden sein und die fünf Öffnungen 268, 270, 272, 274, 276 ansonsten durch das Ventil voneinander gasdicht getrennt sein. Diese erste Situation ist in Figur 10 gezeigt). In einer zweiten Stellung des Ventilkolbens 256 (siehe Figur 11) kann die erste mittlere Öffnung 268 und die dritte Öffnung 272 miteinander über die mittlere Nut 264 gasleitend verbunden sein und die zweite Öffnung 270 und die dazu benachbarte randseitige fünfte Öffnung 274 durch die vorderseitige Nut 262 miteinander gasleitend verbunden sein und die fünf Öffnungen 268, 270, 272, 274, 276 ansonsten durch das Ventil voneinander gasdicht getrennt sein. Der Ventilkolben 256 kann durch die Arbeitskolben 251, 252 von der ersten Stellung in die zweite Stellung überführt werden. Bei Einwirken eines Vakuums 47 am Vakuumanschluss 278 kann in der ersten Stellung des Ventilkolbens 256 der zweite Arbeitsraum 258 evakuiert werden. Gleichzeitig kann der erste Arbeitsraum 257 über die dritte Öffnung 272, die vierte Öffnung 274 und die hintere Nut 266 mit der Umgebung gasdurchlässig verbunden sein, so dass Luft in den ersten Arbeitsraum 257 strömt beziehungsweise vorhanden ist. Durch diese Druckdifferenz kann der erste Arbeitskolben 251 nach hinten gedrückt werden, beziehungsweise der zweite Arbeitskolben 252 nach hinten gezogen werden (in den Figuren 9 bis 11 nach rechts). Wenn der erste Arbeitskolben 251, der mit dem zweiten Arbeitskolben 252 über die Stange 255 verbunden ist, nach hinten gezogen wird und trifft dieser auf den Ventilkolben 256. Der Ventilkolben 256 kann dadurch aus der ersten Stellung herausgestoßen werden und in die zweite Stellung überführt werden. In dieser zweiten Stellung des Ventilkolbens 256 (siehe Figur 11) kann der erste Arbeitsraum 257 evakuiert werden, während er von der Umgebung getrennt ist. Gleichzeitig kann der zweite Arbeitsraum 258 über die zweite Öffnung 270, die fünfte Öffnung 276 und die vordere Nut 262 mit der Umgebung gasdurchlässig verbunden sein, so dass Luft in den zweiten Arbeitsraum 258 strömt und so der zweite Arbeitskolben 252 nach vorne gedrückt werden beziehungsweise der erste Arbeitskolben 251 nach vorne gezogen werden (in den Figuren 9 bis 11 nach links). Hierdurch kann eine periodische lineare Bewegung beziehungsweise Oszillation der Arbeitskolben 251, 252 und des Ventilkolbens 256 erzeugt werden. Diese Bewegung wird durch das Vakuum 47 beziehungsweise den Unterdruck als erster Druck und den umgebenden Luftdruck als zweiter Druck angetrieben. Alternativ kann auch der Vakuumanschluss 278 offen liegen und eine Druckgasquelle mit einem Druck größer als der umgebende Luftdruck an die vierte Öffnung 274 und die fünfte Öffnung 276 angeschlossen werden. Das Funktionsprinzip bleibt dabei das gleiche. Um eine ausreichende Leistung des Differenzdruckmotors zu erzielen, ist ein ausreichender Differenzdruck zwischen dem ersten Druck und dem zweiten Druck sicherzustellen. Zudem kann durch ein Vergrößern der Arbeitsflächen 253, 254 ebenfalls eine Vergrößerung der Leistung des Differenzdruckmotors erreicht werden.

Die Arbeitskolben 251, 252 können über Bolzen 286 mit der Stange 255 verbunden sein. Hierdurch wird der Zusammenbau des Differenzdruckmotors vereinfacht. Die Stange 255 kann sich durch die beiden Arbeitskolben 251, 252 hindurch erstrecken. Hierdurch steht an der Vorderseite des ersten Arbeitskolbens 251 eine erste Antriebsstange 288 vor. Es kann auch vorgesehen sein, dass an der Rückseite des zweiten Arbeitskolbens 252 eine zweite Antriebsstange 290 vorsteht. Auf die zweite Antriebsstange 290 kann aber auch verzichtet werden. Mit den Antriebsstangen 288, 290 können Werkzeuge, wie beispielsweise eine Säge, eine Raspel, eine Bürste oder eine Pumpe (nicht gezeigt) angetrieben werden. Hierzu können an den Enden der Antriebsstangen 288, 290 je ein Befestigungselement 292 angeordnet sein. Das Befestigungselement 292 kann in Form eines Vierkantlochs oder einer Gewindebohrung vorliegen, das mit einem passenden Gegenbefestigungselement an einem Werkzeug verbindbar sein kann.

Der erste Arbeitsraum 253 kann an seiner Vorderseite mit einem Verschluss 293 aus Kunststoff verschlossen sein, der eine Stirnfläche des ersten Arbeitsraums 257 bildet. Die erste Antriebsstange 288 kann durch den Verschluss 293 hindurchgeführt sein. Der Verschluss 293 kann gegen die erste Antriebsstange 288 mit einem Dichtungsring abgedichtet sein, durch den die erste Antriebsstange 288 gleiten kann. Der zweite Arbeitsraum 258 kann an seiner Vorderseite mit dem Verschluss 294 verschlossen sein, der eine Stirnfläche des zweiten Arbeitsraums 258 bildet. Die zweite Antriebsstange 290 kann durch den Verschluss 294 hindurchgeführt sein. Der Verschluss 294 kann gegen die zweite Antriebsstange 290 mit einem Dichtungsring abgedichtet sein, durch den die zweite Antriebsstange 290 gleiten kann.

Der Differenzdruckmotor kann ferner eine Außenhülle 295 aus Kunststoff aufweisen, der die Wandungen 260 umschließt. Die Verschlüsse 293, 294 können mit Schrauben 296 auf die Außenhülle 295 geschraubt sein, wobei zur Abdichtung zwischen den Verschlüssen 93, 94 und der Außenhülle 295 jeweils ein Dichtungsring vorgesehen sein kann. Es kann vorgesehen sein, dass die Öffnungen 268, 270, 272, 274, 276 gegen die Außenhülle 295 mit Dichtungen abgedichtet sind. Die Außenhülle 295 kann auch den Vakuumanschluss 278 ausformen und vorzugsweise auch zum Ausformen von Anschlüssen zur Verbindung der zweiten Öffnung 270 mit der Leitung 282 zu dem zweiten Arbeitsraum 258 und zur Verbindung der dritten Öffnung 272 mit der Leitung 284 zu dem ersten Arbeitsraum 257 verwendet werden.

In der Vakuumleitung 280 und in der Leitung 284 des chirurgischen Antriebssystems nach den Figuren 9 bis 11 kann ein manuell bedienbares Ventilelement 281 angeordnet sein, mit dem die Verbindung zwischen dem Vakuumanschluss 278 und der Vakuumquelle beziehungsweise der Unterdruckquelle unterbrochen werden kann. Das Ventilelement 281 kann einen Ventilkörper 297 aufweisen, der in einem Ventilgehäuse 298 linear verschiebbar gelagert ist. Eine Feder 299 im Ventilgehäuse 298 kann den Ventilkörper 297 in eine geschlossene Stellung drücken. Mit einem Abzug 300 kann der Ventilkörper 297 gegen die Feder 299 in die geöffnete Stellung drücken und so der Differenzdruckmotor zum Laufen gebracht werden.

Auch die Leitung 284, die die dritte Öffnung 272 mit dem ersten Arbeitsraum 257 verbindet, kann also durch das Ventilelement 278 verschlossen und geöffnet werden. Zusätzlich kann eine Ausgleichsleitung 302 vorgesehen sein, die ebenfalls mit dem Ventilelement 281 geöffnet und geschlossen werden kann und die das Vakuum 47 beziehungsweise den Unterdruck in den ersten Arbeitsraum 257 leiten kann. Die Ausgleichsleitung 302 kann über eine Weiche 304 mit der gleichen Vakuumquelle beziehungsweise Unterdruckquelle verbunden sein, wie die Vakuumleitung 280. An der Weiche 304 kann ein Vakuumanschlussstutzen 306 angeordnet sein, an den die Vakuumquelle beziehungsweise die Unterdruckquelle angeschlossen werden kann, vorzugsweise über einen flexiblen Schlauch (nicht gezeigt).

Das Ventilelement 281 kann also ein Dreiwege-Ventilelement sein. Im entspannten Ruhezustand des Ventilelements 281 (siehe Figur 9) kann die Vakuumleitung 280 und die Leitung 284 zwischen dem ersten Arbeitsraum 257 und der zweiten Öffnung 272 durch den Ventilkörper 297 verschlossen sein, während die Ausgleichsleitung 302 geöffnet ist. Dadurch kann, wenn ein Vakuum 47 an der Ausgleichleitung 302 anliegt, der erste Arbeitskolben 251 nach vorne gezogen werden. In dieser Ruhestellung befindet sich der Ventilkolben 256 dann in der ersten Stellung. Wenn der Abzug 300 bedient wird, kann die Ausgleichsleitung 302 geschlossen werden. Gleichzeitig kann so die die Leitung 284 zwischen dem ersten Arbeitsraum 257 und der zweiten Öffnung 272 und die Vakuumleitung 280 geöffnet werden. Da durch diesen Aufbau sichergestellt ist, dass der Ventilkolben 256 sich in der ersten Stellung befindet, wenn das Ventilelement 281 bedient wird, wird verhindert, dass der Differenzdruckmotor in einem Totpunkt zum Stehen kommt und nicht wieder anspringen kann.

Die gleiche Starthilfe oder Totpunktverhinderung kann auch bei einem der anderen erfindungsgemäßen Differenzdruckmotoren nach den Figuren 1 bis 5 oder 6 und 7 oder 8 verwendet werden. Die Starthilfe nach dem vierten Ausführungsbeispiel benötigt keine Feder.

Alle Teile der beispielhaften Differenzdruckmotoren sowie des medizinischen Antriebssystems können aus Kunststoff gefertigt sein. Damit kann der Differenzdruckmotor als kostengünstiges Wegwerfprodukt für medizinische Anwendungen verwendet und im Anschluss an seine Verwendung hygienisch verbrennt werden. Dadurch lässt sich eine Kontamination des medizinischen Personals beim Desinfizieren und von Patienten aufgrund der Vermeidung einer Mehrfachverwendung ausschließen.

Alternativ zu der Säge 102 können auch andere Werkzeuge wie Raspeln oder Bürsten (nicht gezeigt) mit den Befestigungselementen 42, 92, 192, 292 verbunden werden oder es kann auch eine Flüssigkeitspumpe angeschlossen und mit dem Differenzdruckmotor betrieben werden, so dass mit dem Differenzdruckmotor bei jedem Bewegungszyklus der Stange 5, 55, 155, 255 beziehungsweise der ersten Antriebsstange 38, 88, 188, 288 und/oder der zweiten Antriebsstange 40, 90, 190, 290 ein Sprühstoß mit einer medizinischen Spülflüssigkeit erzeugt wird. Auf diese Weise kann ein erfindungsgemäßes Lavage-System realisiert werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51, 151, 251: Erster Arbeitskolben
- 2, 52, 152, 252: Zweiter Arbeitskolben
- 3, 53, 153, 253: Arbeitsfläche
- 4, 54, 154, 254: Arbeitsfläche
- 5, 55, 155, 255: Stange
- 6, 56, 156, 256: Ventilkolben
- 7, 57, 157, 257: Erster Arbeitsraum
- 8, 58, 158, 258: Zweiter Arbeitsraum
- 9, 59, 159, 259: Ventilraum
- 10, 60, 160, 260: Wandung
- 12, 62, 162, 262: Nut
- 14, 64, 164, 264: Nut
- 16, 66, 166, 266: Nut
- 18, 68, 168, 268: Erste Öffnung
- 20, 70, 170, 270: Zweite Öffnung
- 22, 72, 172, 272: Dritte Öffnung
- 24, 74, 174, 274: Vierte Öffnung
- 26, 76, 176, 276: Fünfte Öffnung
- 28, 78, 278: Vakuumanschluss
- 30, 80, 280: Vakuumleitung
- 32, 82, 182, 282: Leitung
- 34, 84, 184, 284: Leitung
- 36, 86, 186, 286: Bolzen
- 38, 88, 188, 288: Antriebsstange
- 40, 90, 190, 290: Antriebsstange
- 42, 92, 192, 292: Befestigungselement
- 43, 93, 193, 293: Verschluss mit Stirnfläche
- 44, 94, 194, 294: Verschluss mit Stirnfläche
- 45, 95, 195, 295: Außenhülle
- 46, 96, 196, 296: Schraube
- 47: Vakuum
- 61: Stopfen
- 81, 281: Ventilelement
- 91: Federelement
- 97, 297: Ventilkörper
- 98, 298: Ventilgehäuse
- 99, 299: Feder
- 100, 300: Abzug
- 102: Säge
- 104: Gehäuse
- 106: Griff
- 178: Druckgasanschluss
- 180: Druckgasleitung
- 197: Druckgas
- 302: Ausgleichsleitung
- 304: Weiche
- 306: Vakuumanschlussstutzen

## Patentansprüche

1. Differenzdruckmotor aufweisend
zwei Arbeitskolben (1, 2, 51, 52, 151, 152, 251, 252) mit je einer Arbeitsfläche (3, 4, 53, 54, 153, 154, 253, 254),
eine Stange (5, 55, 155, 255), die die Arbeitskolben (1, 2, 51, 52, 151, 152, 251, 252) miteinander verbindet und voneinander beabstandet,
einen Hohlraum, der zumindest bereichsweise zylindrisch ist, wobei die Arbeitskolben (1, 2, 51, 52, 151, 152, 251, 252) und die Stange (5, 55, 155, 255) beweglich in dem Hohlraum angeordnet sind, wobei in Wandungen (10, 60, 160, 260) des Hohlraums fünf durchgehende Öffnungen (18, 20, 22, 24, 26, 68, 70, 72, 74, 76, 168, 170, 172, 174, 176, 268, 270, 272, 274, 276) angeordnet sind,
einen Ventilkolben (6, 56, 156, 256), wobei der Ventilkolben (6, 56, 156, 256) zwischen den Arbeitskolben (1, 2, 51, 52, 151, 152, 251, 252) gegen die Arbeitskolben (1, 2, 51, 52, 151, 152, 251, 252) beweglich angeordnet ist und der Ventilkolben (6, 56, 156, 256) durch Stöße der Arbeitskolben (1, 2, 51, 52, 151, 152, 251, 252) gegen den Ventilkolben (6, 56, 156, 256) anzutreiben ist und in dem zylindrischen Hohlraum beweglich ist, wobei der Ventilkolben (6, 56, 156, 256) mit den fünf durchgehenden Öffnungen (18, 20, 22, 24, 26, 68, 70, 72, 74, 76, 168, 170, 172, 174, 176, 268, 270, 272, 274, 276) ein Ventil bildet, mit dem eine abwechselnde Beaufschlagung der Arbeitsflächen (3, 4, 53, 54, 153, 154, 253, 254) der Arbeitskolben (1, 2, 51, 52, 151, 152, 251, 252) mit einem ersten Druck und einem zweiten Druck steuerbar ist, wenn der erste Druck und der zweite Druck an drei der fünf durchgehenden Öffnungen (18, 20, 22, 24, 26, 68, 70, 72, 74, 76, 168, 170, 172, 174, 176, 268, 270, 272, 274, 276) angelegt sind, so dass eine periodische Bewegung der Arbeitskolben (1, 2, 51, 52, 151, 152, 251, 252) und der Stange (5, 55, 155, 255) im Hohlzylinder entsteht, die eine periodische Bewegung des Ventilkolbens (6, 56, 156, 256) antreibt.

2. Differenzdruckmotor nach Anspruch 1, **dadurch gekennzeichnet, dass**
die Arbeitskolben (1, 2, 51, 52, 151, 152, 251, 252) den Hohlraum in zumindest drei voneinander getrennte Bereiche unterteilt, und
der Hohlraum einen ersten Arbeitsraum (7, 57, 157, 257) und einen zweiten Arbeitsraum (8, 58, 158, 258) aufweist, in denen die Arbeitskolben (1, 2, 51, 52, 151, 152, 251, 252) beweglich angeordnet sind, und einen zwischen den Arbeitsräumen (7, 8, 57, 58, 157, 158, 257, 258) angeordneten Ventilraum (9, 59, 159, 259) aufweist, wobei die fünf durchgehenden Öffnungen (18, 20, 22, 24, 26, 68, 70, 72, 74, 76, 168, 170, 172, 174, 176, 268, 270, 272, 274, 276) in jeder Stellung der Arbeitskolben (1, 2, 51, 52, 151, 152, 251, 252) im Ventilraum (9, 59, 159, 259) angeordnet sind, und/oder der axiale Abstand einer ersten Stirnfläche des Ventilkolbens (6, 56, 156, 256) zur Rückseite des benachbarten Arbeitskolbens (1, 2, 51, 52, 151, 152, 251, 252) kleiner oder gleich ist, wie der Abstand der Arbeitsfläche (3, 4, 53, 54, 153, 154, 253, 254) dieses Arbeitskolbens (1, 2, 51, 52, 151, 152, 251, 252) zu einer ersten Stirnfläche des Hohlraums, und der axiale Abstand einer zweiten Stirnfläche des Ventilkolbens (6, 56, 156, 256) zur Rückseite des benachbarten Arbeitskolbens (1, 2, 51, 52, 151, 152, 251, 252) kleiner oder gleich ist, wie der Abstand der Arbeitsfläche (3, 4, 53, 54, 153, 154, 253, 254) dieses Arbeitskolbens (1, 2, 51, 52, 151, 152, 251, 252) zu einer zweiten, der ersten Stirnfläche gegenüberliegenden Stirnfläche des Hohlraums.

3. Differenzdruckmotor nach einem der vorangehenden Ansprüche, **dadurch gekennzeichnet, dass**
die Arbeitskolben (1, 2, 51, 52, 151, 152, 251, 252) und die Stange (5, 55, 155, 255) linear beweglich, insbesondere axial beweglich bezogen auf eine Symmetrieachse der Arbeitskolben (1, 2, 51, 52, 151, 152, 251, 252) und/oder der Stange (5, 55, 155, 255), in dem zumindest bereichsweise zylindrischen Hohlraum angeordnet sind, und/oder der Ventilkolben (6, 56, 156, 256) mit den fünf Öffnungen (18, 20, 22, 24, 26, 68, 70, 72, 74, 76, 168, 170, 172, 174, 176, 268, 270, 272, 274, 276) ein 2/5-Wegeventil bildet, bevorzugt ein 2/5-Wege-lmpuls-Ventil bildet, und/oder
das Ventil eine alternierende Beaufschlagung der Arbeitskolben (1, 2, 51, 52, 151, 152, 251, 252) mit einem Vakuum (47) oder einem Druckgas (197) und mit umgebender Atmosphäre steuert.

4. Differenzdruckmotor nach einem der vorangehenden Ansprüche, **dadurch gekennzeichnet, dass**
der Ventilkolben (6, 56, 156, 256) eine Hülse mit drei umlaufenden Nuten (12, 14, 16, 62, 64, 66, 162, 164, 166, 262, 264, 266) ist, die durch umlaufende Stege voneinander getrennt sind, wobei die Nuten (12, 14, 16, 62, 64, 66, 162, 164, 166, 262, 264, 266) zumindest so breit sind, wie der auf die Hülse bezogene axiale Abstand zweier axial benachbarter Öffnungen (18, 20, 22, 24, 26, 68, 70, 72, 74, 76, 168, 170, 172, 174, 176, 268, 270, 272, 274, 276) der fünf Öffnungen (18, 20, 22, 24, 26, 68, 70, 72, 74, 76, 168, 170, 172, 174, 176, 268, 270, 272, 274, 276), so dass bei geeigneter Stellung der Hülse im Hohlraum zwei axial benachbarte Öffnungen (18, 20, 22, 24, 26, 68, 70, 72, 74, 76, 168, 170, 172, 174, 176, 268, 270, 272, 274, 276) in die gleiche Nut (12, 14, 16, 62, 64, 66, 162, 164, 166, 262, 264, 266) der Hülse im Hohlraum münden, und wobei bevorzugt die Stege zumindest in axialer Richtung so breit sind wie die Mündungen in den Hohlraum der beiden Öffnungen (20, 22, 70, 72, 170, 172, 270, 272) der fünf Öffnungen (18, 20, 22, 24, 26, 68, 70, 72, 74, 76, 168, 170, 172, 174, 176, 268, 270, 272, 274, 276), die zu den äußersten Öffnungen (24, 26, 74, 76, 174, 176, 274, 276) benachbart sind, und/oder
die Arbeitsflächen (3, 4, 53, 54, 153, 154, 253, 254) der Arbeitskolben (1, 2, 51, 52, 151, 152, 251, 252) voneinander abgewandt ausgerichtet sind, und/oder der Ventilkolben (6, 56, 156, 256) auf der Stange (5, 55, 155, 255) axial verschiebbar angeordnet ist.

5. Differenzdruckmotor nach einem der vorangehenden Ansprüche, **dadurch gekennzeichnet, dass**
eine mittlere Öffnung (18, 68, 168, 268) der fünf Öffnungen (18, 20, 22, 24, 26, 68, 70, 72, 74, 76, 168, 170, 172, 174, 176, 268, 270, 272, 274, 276) mit einem Vakuumanschluss (28, 78, 278) oder einem Druckluftanschluss (178) verbunden ist und zwei äußere Öffnungen (24, 26, 74, 76, 174, 176, 274, 276) nach außen zur Umgebung des Differenzdruckmotors offen sind oder
zwei äußere Öffnungen (24, 26, 74, 76, 174, 176, 274, 276) der fünf Öffnungen (18, 20, 22, 24, 26, 68, 70, 72, 74, 76, 168, 170, 172, 174, 176, 268, 270, 272, 274, 276) mit einem Vakuumanschluss (28, 78, 278) oder einem Druckluftanschluss (178) verbunden sind und eine mittele Öffnung (18, 68, 168, 268) nach außen zur Umgebung des Differenzdruckmotors offen sind,
wobei in beiden Fällen eine zu einer äußeren Öffnung (24, 26, 74, 76, 174, 176, 274, 276) benachbarte Öffnung (20, 22, 70, 72, 170, 172, 270, 272) der fünf Öffnungen (18, 20, 22, 24, 26, 68, 70, 72, 74, 76, 168, 170, 172, 174, 176, 268, 270, 272, 274, 276) mit einem durch einen ersten Arbeitskolben (1, 51, 151, 251) der zwei Arbeitskolben (1, 2, 51, 52, 151, 152, 251, 252) begrenzten ersten Arbeitsraum (7, 57, 157, 257) druckleitend verbunden ist und eine andere zu einer äußeren Öffnung (24, 26, 74, 76, 174, 176, 274, 276) benachbarte Öffnung (20, 22, 70, 72, 170, 172, 270, 272) mit einem durch einen zweiten Arbeitskolben (2, 52, 152, 252) der zwei Arbeitskolben (1, 2, 51, 52, 151, 152, 251, 252) begrenzten zweiten Arbeitsraum (8, 58, 158, 258) druckleitend verbunden ist.

6. Differenzdruckmotor nach einem der vorangehenden Ansprüche, **dadurch gekennzeichnet, dass**
bei Anlegen eines Vakuums (47) an einem dafür vorgesehenen Vakuumanschluss (28, 78, 278) des Differenzdruckmotors die beiden Arbeitskolben (1, 2, 51, 52, 151, 152, 251, 252) durch periodische Wechsel der Einwirkung von Vakuum und der umgebenden Atmosphäre eine oszillierende Bewegung der Stange (5, 55, 155, 255) bewirken oder
bei Einspeisen von Druckgas (197) an einem dafür vorgesehenen Druckgasanschluss (178) des Differenzdruckmotors die beiden Arbeitskolben (1, 2, 51, 52, 151, 152, 251, 252) durch periodische Wechsel der Einwirkung von Druckluft und der umgebenden Atmosphäre eine oszillierende Bewegung der Stange (5, 55, 155, 255) bewirken, wobei in beiden Fällen die Arbeitskolben (1, 2, 51, 52, 151, 152, 251, 252) periodisch den Ventilkolben (6, 56, 156, 256) beidseitig anstoßen und so eine axiale periodische Bewegung des Ventilkolbens (6, 56, 156, 256) verursachen, wodurch ein Umschalten des Ventils erfolgt.

7. Differenzdruckmotor nach einem der vorangehenden Ansprüche, **dadurch gekennzeichnet, dass**
in einer ersten Stellung des Ventilkolbens (6, 56, 156, 256) eine erste mittlere Öffnung (18, 68, 168, 268) der fünf Öffnungen (18, 20, 22, 24, 26, 68, 70, 72, 74, 76, 168, 170, 172, 174, 176, 268, 270, 272, 274, 276) und eine zur ersten Öffnung (18, 68, 168, 268) benachbarte zweite Öffnung (20, 70, 170, 270) der fünf Öffnungen (18, 20, 22, 24, 26, 68, 70, 72, 74, 76, 168, 170, 172, 174, 176, 268, 270, 272, 274, 276) miteinander über das Ventil gasleitend verbunden sind und eine der zweiten Öffnung (20, 70, 170, 270) bezogen auf die erste Öffnung (18, 68, 168, 268) gegenüberliegende dritte Öffnung (22, 72, 172, 272) der fünf Öffnungen (18, 20, 22, 24, 26, 68, 70, 72, 74, 76, 168, 170, 172, 174, 176, 268, 270, 272, 274, 276) und eine dazu benachbarte randseitige vierte Öffnung (24, 74, 174, 274) der fünf Öffnungen (18, 20, 22, 24, 26, 68, 70, 72, 74, 76, 168, 170, 172, 174, 176, 268, 270, 272, 274, 276) durch das Ventil miteinander gasleitend verbunden sind und die fünf Öffnungen (18, 20, 22, 24, 26, 68, 70, 72, 74, 76, 168, 170, 172, 174, 176, 268, 270, 272, 274, 276) ansonsten im Hohlraum durch das Ventil voneinander gasdicht getrennt sind und
in einer zweiten Stellung des Ventilkolbens (6, 56, 156, 256) die erste mittlere Öffnung (18, 68, 168, 268) und die dritte Öffnung (22, 72, 172, 272) miteinander über das Ventil gasleitend verbunden sind und die zweite Öffnung (20, 70, 170, 270) und eine dazu benachbarte randseitige fünfte (26, 76, 176, 276) Öffnung der fünf Öffnungen (18, 20, 22, 24, 26, 68, 70, 72, 74, 76, 168, 170, 172, 174, 176, 268, 270, 272, 274, 276) durch das Ventil miteinander gasleitend verbunden sind und die fünf Öffnungen (18, 20, 22, 24, 26, 68, 70, 72, 74, 76, 168, 170, 172, 174, 176, 268, 270, 272, 274, 276) ansonsten im Hohlraum durch das Ventil voneinander gasdicht getrennt sind, und
bevorzugt der Ventilkolben (6, 56, 156, 256) von der ersten Stellung des Ventilkolbens (6, 56, 156, 256) in die zweite Stellung des Ventilkolbens (6, 56, 156, 256) durch eine Impulsübertragung eines ersten Arbeitskolbens (1, 51, 151, 251) der zwei Arbeitskolben (1, 2, 51, 52, 151, 152, 251, 252) auf den Ventilkolben (6, 56, 156, 256) überführbar ist und der Ventilkolben (6, 56, 156, 256) von der zweiten Stellung des Ventilkolbens (6, 56, 156, 256) in die erste Stellung des Ventilkolbens (6, 56, 156, 256) durch eine Impulsübertragung eines zweiten Arbeitskolbens (2, 52, 152, 252) der zwei Arbeitskolben (1, 2, 51, 52, 151, 152, 251, 252) auf den Ventilkolben (6, 56, 156, 256) überführbar ist.

8. Differenzdruckmotor nach Anspruch 7, **dadurch gekennzeichnet, dass**
der erste Arbeitskolben (1, 51, 151, 251) in der ersten Stellung des Ventilkolbens (6, 56, 156, 256) aufgrund eines auf der Arbeitsfläche (3, 53, 153, 253) des ersten Arbeitskolbens (1, 51, 151, 251) lastenden Drucks in Richtung des Ventilkolbens (6, 56, 156, 256) beschleunigt wird und der zweite Arbeitskolben (2, 52, 152, 252) in der zweiten Stellung des Ventilkolbens (6, 56, 156, 256) aufgrund eines auf der Arbeitsfläche (4, 54, 154, 254) des zweiten Arbeitskolbens (2, 52, 152, 252) lastenden Drucks in Richtung des Ventilkolbens (6, 56, 156, 256) beschleunigt wird, und/oder
ein erster Arbeitsraum (7, 57, 157, 257), der durch den ersten Arbeitskolben (1, 51, 151, 251) begrenzt ist, gasleitend mit der zweiten Öffnung (20, 70, 170, 270) verbunden ist und ein zweiter Arbeitsraum (8, 58, 158, 258), der durch den zweiten Arbeitskolben (2, 52, 152, 252) begrenzt ist, gasleitend mit der dritten Öffnung (22, 72, 172, 272) verbunden ist.

9. Differenzdruckmotor nach einem der vorangehenden Ansprüche, **dadurch gekennzeichnet, dass**
der Differenzdruckmotor ein Federelement (91) aufweist, das sich an einer geschlossenen Außenseite einer Stirnfläche eines den Hohlraum begrenzenden Gehäuses abstützt und das mit der Stange (5, 55, 155, 255) verbunden ist, so dass ohne eine Krafteinwirkung durch einen Differenzdruck das Federelement (91) die Stange (5, 55, 155, 255) maximal aus dem Hohlraum zieht, und/oder
die zwei Arbeitskolben (1, 2, 51, 52, 151, 152, 251, 252) einen Durchmesser von größer 10 mm, bevorzugt von größer 20 mm und ganz besonders bevorzugt von größer 30 mm haben.

10. Differenzdruckmotor nach einem der vorangehenden Ansprüche, **dadurch gekennzeichnet, dass**
zumindest eine Antriebsstange (38, 40, 88, 90, 188, 190, 288, 290) an einer äußeren Seite wenigstens eines der zwei Arbeitskolben (1, 2, 51, 52, 151, 152, 251, 252) angeordnet ist, wobei die zumindest eine Antriebsstange (38, 40, 88, 90, 188, 190, 288, 290) durch eine Vorderseite und/oder einer Rückseite eines den Hohlraum begrenzenden Gehäuses hindurch aus dem Gehäuse herausragt und die zumindest eine Antriebsstange (38, 40, 88, 90, 188, 190, 288, 290) in einer Führung in der Vorderseite und/oder der Rückseite des Gehäuses beweglich gelagert ist, wobei vorzugsweise die Stange (5, 55, 155, 255), die die beiden Arbeitskolben (1, 2, 51, 52, 151, 152, 251, 252) verbindet, durch zumindest einen der Arbeitskolben (1, 2, 51, 52, 151, 152, 251, 252) hindurch geführt ist und dort die zumindest eine Antriebsstange (38, 40, 88, 90, 188, 190, 288, 290) bildet und/oder
vorzugsweise an der Vorderseite der zumindest einen Antriebsstange (38, 40, 88, 90, 188, 190, 288, 290) ein Befestigungselement (42, 92, 192, 292), insbesondere ein Gewinde, angeordnet ist, über das ein Werkzeug, wie eine Säge (102), eine Raspel oder eine Bürste, mit einem zum Befestigungselement (42, 92, 192, 292) passenden Gegenbefestigungselement, insbesondere einem zum Gewinde passenden Gegengewinde, an der zumindest einen Antriebsstange (38, 40, 88, 90, 188, 190, 288, 290) befestigbar ist.

11. Differenzdruckmotor nach einem der vorangehenden Ansprüche, **dadurch gekennzeichnet, dass**
ein den Hohlraum begrenzendes Gehäuse, die zwei Arbeitskolben (1, 2, 51, 52, 151, 152, 251, 252), der Ventilkolben (6, 56, 156, 256) und die Stange (5, 55, 155, 255) aus einem Kunststoff gefertigt sind, insbesondere aus einem thermoplastischen Kunststoff gefertigt sind, vorzugsweise durch Spritzgießen geformt sind, und/oder die geometrische Abmessung des Ventilkolbens (6, 56, 156, 256) in Richtung einer Verbindungslinie zwischen den beiden Arbeitskolben (1, 2, 51, 52, 151, 152, 251, 252) kleiner ist als der Abstand der beiden Arbeitskolben (1, 2, 51, 52, 151, 152, 251, 252), wobei bevorzugt die geometrische Abmessung des Ventilkolbens (6, 56, 156, 256) in Richtung der Verbindungslinie zwischen den beiden Arbeitskolben (1, 2, 51, 52, 151, 152, 251, 252) maximal 50% kleiner ist als der Abstand zwischen den beiden Arbeitskolben (1, 2, 51, 52, 151, 152, 251, 252), besonders bevorzugt maximal 10% kleiner ist als der Abstand zwischen den beiden Arbeitskolben (1, 2, 51, 52, 151, 152, 251, 252), und/oder
der Differenzdruckmotor eine Starthilfe aufweist, die verhindert, dass der Differenzdruckmotor bei einer Unterbrechung der Zufuhr des Arbeitsmediums in einem Totpunkt anhält, aus dem heraus er nicht mehr von alleine startet, wenn die Zufuhr des Arbeitsmediums in den Differenzdruckmotor wieder erfolgt, wobei vorzugsweise die Starthilfe den Ventilkolben (6, 56, 156, 256) in eine Stellung überführt, in der durch das Arbeitsmedium eine Druckdifferenz zwischen den Arbeitsflächen (3, 4, 53, 54, 153, 154, 253, 254) der Arbeitskolben (1, 2, 51, 52, 151, 152, 251, 252) erzeugbar ist.

12. Chirurgisches Antriebssystem aufweisend einen Differenzdruckmotor nach einem der vorangehenden Ansprüche sowie ein Ventilelement (81, 281), insbesondere ein manuell bedienbares Ventilelement (81, 281), wobei das Ventilelement (81, 281) in einer Leitung angeordnet ist, die mit einer der fünf Öffnungen (18, 20, 22, 24, 26, 68, 70, 72, 74, 76, 168, 170, 172, 174, 176, 268, 270, 272, 274, 276) oder mit zwei der fünf Öffnungen (18, 20, 22, 24, 26, 68, 70, 72, 74, 76, 168, 170, 172, 174, 176, 268, 270, 272, 274, 276) verbunden ist und die mit einer Unterdruckquelle oder einem Druckgasreservoir oder einer Pumpe verbindbar oder verbunden ist, so dass mit dem Ventilelement (81, 281) die Verbindung zur Unterdruckquelle, dem Druckgasreservoir oder der Pumpe unterbrechbar und/oder der Druck an der einen Öffnung (18, 68, 168, 268) der fünf Öffnungen (18, 20, 22, 24, 26, 68, 70, 72, 74, 76, 168, 170, 172, 174, 176, 268, 270, 272, 274, 276) oder der zwei Öffnungen (24, 26, 74, 76, 174, 176, 274, 276) der fünf Öffnungen (18, 20, 22, 24, 26, 68, 70, 72, 74, 76, 168, 170, 172, 174, 176, 268, 270, 272, 274, 276) einstellbar ist, wobei
vorzugsweise das chirurgische Antriebssystem einen Griff (106) aufweist, mit dem es in einer Hand haltbar ist und das Ventilelement (81, 281) mit einem Abzug (100, 300) am Griff (106) bedienbar ist.

13. Medizinisches Lavage-System zum Debridement von Weichgewebe und/oder Knochengewebe aufweisend einen Differenzdruckmotor nach einem der Ansprüche 1 bis 11 oder ein chirurgisches Antriebssystem nach Anspruch 12 oder
medizinische Vorrichtung zum Bürsten, Raspeln oder Sägen von Weichgewebe und/oder Knochengewebe aufweisend einen Differenzdruckmotor nach einem der Ansprüche 1 bis 11 oder ein chirurgisches Antriebssystem nach Anspruch 12.

14. Verfahren zum Betreiben eines Differenzdruckmotors, bei dem ein erster Arbeitskolben (1, 51, 251) und ein zweiter Arbeitskolben (2, 52, 252) über eine Stange (5, 55, 255) verbunden sind und linear in einem Hohlraum oszillieren, wobei das Verfahren die folgenden Schritte umfasst:
A) ein Ventilkolben (6, 56, 256), der zwischen dem ersten Arbeitskolben (1, 51, 251) und dem zweiten Arbeitskolben (2, 52, 252) im Hohlraum angeordnet ist, befindet sich in einer ersten Stellung;
B) Bereitstellen einer Verbindung zwischen einem Vakuumanschluss (28, 78, 278) zu einem ersten Arbeitsraum (7, 57, 257) und einer Verbindung zwischen einem zweiten Arbeitsraum (8, 58, 258) und der Umgebung des Differenzdruckmotors in der ersten Stellung des Ventilkolbens (6, 56, 256) durch den Ventilkolben (6, 56, 256), wobei der erste Arbeitsraum (7, 57, 257) von dem ersten Arbeitskolben (1, 51, 251) begrenzt ist und der zweite Arbeitsraum (8, 58, 258) von dem zweiten Arbeitskolben (2, 52, 252) begrenzt ist;
C) Evakuieren von Gas aus dem ersten Arbeitsraum (7, 57, 257) und dadurch Bewegen des ersten Arbeitskolbens (1, 51, 251) und des zweiten Arbeitskolbens (2, 52, 252) in dem Hohlraum;
D) Anstoßen des Ventilkolbens (6, 56, 256) mit dem zweiten Arbeitskolben (2, 52, 252), so dass der Ventilkolben (6, 56, 256) in eine zweite Stellung überführt wird;
E) Bereitstellen einer Verbindung zwischen dem Vakuumanschluss (28, 78, 278) zu dem zweiten Arbeitsraum (8, 58, 258) und einer Verbindung zwischen dem ersten Arbeitsraum (7, 57, 257) und der Umgebung des Differenzdruckmotors in der zweiten Stellung des Ventilkolbens (6, 56, 256) durch den Ventilkolben (6, 56, 256);
F) Evakuieren von Gas aus dem zweiten Arbeitsraum (8, 58, 258) und Einströmen von Umgebungsluft in den ersten Arbeitsraum (7, 57, 257) und dadurch umgekehrtes Bewegen des ersten Arbeitskolbens (1, 51, 251) und des zweiten Arbeitskolbens (2, 52, 252) in dem Hohlraum;
G) Anstoßen des Ventilkolbens (6, 56, 256) mit dem ersten Arbeitskolben (1, 51, 251), so dass der Ventilkolben (6, 56, 256) in die erste Stellung überführt wird.

15. Verfahren zum Betreiben eines Differenzdruckmotors, bei dem ein erster Arbeitskolben (151) und ein zweiter Arbeitskolben (152) über eine Stange (155) verbunden sind und linear in einem Hohlraum oszillieren, wobei das Verfahren die folgenden Schritte umfasst:
A) ein Ventilkolben (156), der zwischen dem ersten Arbeitskolben (151) und dem zweiten Arbeitskolben (152) im Hohlraum angeordnet ist, befindet sich in einer ersten Stellung;
B) Bereitstellen einer Verbindung zwischen einem Druckgasanschluss (178) zu einem ersten Arbeitsraum (157) und einer Verbindung zwischen einem zweiten Arbeitsraum (158) und der Umgebung des Differenzdruckmotors in der ersten Stellung des Ventilkolbens (156) durch den Ventilkolben (156), wobei der erste Arbeitsraum (157) von dem ersten Arbeitskolben (151) begrenzt ist und der zweite Arbeitsraum (158) von dem zweiten Arbeitskolben (152) begrenzt ist;
C) Erhöhen des Gasdrucks in dem ersten Arbeitsraum (157) und dadurch Bewegen des ersten Arbeitskolbens (151) und des zweiten Arbeitskolbens (152) in dem Hohlraum;
D) Anstoßen des Ventilkolbens (156) mit dem ersten Arbeitskolben (151), so dass der Ventilkolben (156) in eine zweite Stellung überführt wird;
E) Bereitstellen einer Verbindung zwischen dem Druckgasanschluss (178) zu dem zweiten Arbeitsraum (158) und einer Verbindung zwischen dem ersten Arbeitsraum (157) und der Umgebung des Differenzdruckmotors in der zweiten Stellung des Ventilkolbens (156) durch den Ventilkolben (156);
F) Erhöhen des Gasdrucks in dem zweiten Arbeitsraum (158) und Ausströmen von Druckgas aus dem ersten Arbeitsraum (157) und dadurch umgekehrtes Bewegen des ersten Arbeitskolbens (151) und des zweiten Arbeitskolbens (152) in dem Hohlraum;
G) Anstoßen des Ventilkolbens (156) mit dem zweiten Arbeitskolben (152), so dass der Ventilkolben (156) in die erste Stellung überführt wird.

16. Verfahren nach Anspruch 14 oder 15, **dadurch gekennzeichnet, dass**
das Verfahren mit einem Differenzdruckmotor nach einem der Ansprüche 1 bis 11 oder mit einem chirurgischen Antriebssystem nach Anspruch 12 oder mit einem Lavage-System oder einer medizinischen Vorrichtung zum Bürsten, Raspeln oder Sägen von Weichgewebe und/oder Knochengewebe nach Anspruch 13 durchgeführt wird.

17. Verfahren nach einem der Ansprüche 15 oder 16, **dadurch gekennzeichnet, dass**
der Vakuumanschluss oder der Druckgasanschluss durch eine erste mittlere Öffnung (18, 68, 168, 268) in einen Ventilraum (9, 59, 159, 259) münden, in dem sich der Ventilkolben (9, 59, 159, 259) bewegt, wobei neben der ersten mittleren Öffnung (18, 68, 168, 268) eine zweite Öffnung (20, 70, 170, 270) angeordnet ist, die den Ventilraum (9, 59, 159, 259) mit dem ersten Arbeitsraum (7, 57, 157, 257) verbindet, neben der ersten mittleren Öffnung (18, 68, 168, 268) eine dritte Öffnung (22, 72, 172, 272) angeordnet ist, die den Ventilraum (9, 59, 159, 259) mit dem zweiten Arbeitsraum (8, 58, 158, 258) verbindet, neben der zweiten Öffnung (20, 70, 170, 270) eine vierte äußere Öffnung (24, 74, 174, 274) angeordnet ist, die den Ventilraum (9, 59, 159, 259) mit der Umgebung des Differenzdruckmotors verbindet, und neben der dritten Öffnung (22, 72, 172, 272) eine fünfte äußere Öffnung (26, 76, 176, 276) angeordnet ist, die den Ventilraum (9, 59, 159, 259) mit der Umgebung des Differenzdruckmotors verbindet, wobei in dem Ventilkolben (6, 56, 156, 256) drei Verbindungen, insbesondere drei Nuten (12, 14, 16, 62, 64, 66, 162, 164, 166, 262, 264, 266), angeordnet sind, so dass in der ersten Stellung des Ventilkolbens (6, 56, 156, 256) die mittlere erste Öffnung (18, 68, 168, 268) mit der zweiten Öffnung (20, 70, 170, 270) gasdurchlässig verbunden wird und die dritte Öffnung (22, 72, 172, 272) mit der fünften Öffnung (26, 76, 176, 276) gasdurchlässig verbunden wird und in der zweiten Stellung des Ventilkolbens (6, 56, 156, 256) die mittlere erste Öffnung (18, 68, 168, 268) mit der dritten Öffnung (22, 72, 172, 272) gasdurchlässig verbunden wird und die zweite Öffnung (20, 70, 170, 270) mit der vierten Öffnung (24, 74, 174, 274) gasdurchlässig verbunden wird, wobei vorzugsweise die fünf Öffnungen (18, 20, 22, 24, 26, 68, 70, 72, 74, 76, 168, 170, 172, 174, 176, 268, 270, 272, 274, 276) ansonsten voneinander durch den Ventilkolben (6, 56, 156, 256) getrennt werden.

18. Verfahren nach einem der Ansprüche 15 bis 17, **dadurch gekennzeichnet, dass**
nach Schritt G) eine Wiederholung der Schritte B) bis G) erfolgt, solange am Vakuumanschluss (28, 78, 278) ein Vakuum (47) oder ein Unterdruck anliegt oder solange am Druckgasanschluss (178) ein Überdruck anliegt oder ein Druckgas (197) eingespeist wird, und/oder
mit der Bewegung des ersten Arbeitskolbens (1, 51, 151, 251) und des zweiten Arbeitskolbens (2, 52, 152, 252) ein Werkzeug oder eine Pumpe angetrieben wird, insbesondere ein medizinisches Werkzeug, wie eine Säge (102), eine Raspel oder eine Bürste, oder ein Lavage-System angetrieben wird, und/oder
der Differenzdruckmotor durch Öffnen eines Ventilelements (81, 281) in einer Vakuumleitung (30, 80, 280) am Vakuumanschluss (28, 78, 278) oder in einer Druckgasleitung (180) am Druckgasanschluss (178) angeschaltet und durch Schließen des Ventilelements (81, 281) ausgeschaltet wird.
